(19) Europäisches Patentamt
European Patent Office
Office européen des brevets

(11) **EP 4 706 497 A1**

(12) **EUROPÄISCHE PATENTANMELDUNG**

(43) Veröffentlichungstag:
**11.03.2026 Patentblatt 2026/11**

(21) Anmeldenummer: **25197318.6**

(22) Anmeldetag: **21.08.2025**

(51) Internationale Patentklassifikation (IPC):
*A61B 1/06* (2006.01)     *A61B 1/07* (2006.01)
*G02B 23/24* (2006.01)     *G02B 27/10* (2006.01)
*G02B 27/14* (2006.01)

(52) Gemeinsame Patentklassifikation (CPC):
**A61B 1/063; A61B 1/0638; A61B 1/0646;**
**A61B 1/0669; A61B 1/0684; A61B 1/07;**
**G02B 23/2461; G02B 23/2469; G02B 27/1006;**
**G02B 27/141; G02B 27/143; G02B 27/144;**
**G02B 27/145**

(84) Benannte Vertragsstaaten:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Benannte Erstreckungsstaaten:
**BA**
Benannte Validierungsstaaten:
**GE KH LA MA MD TN**

(30) Priorität: **06.09.2024 DE 102024125695**

(71) Anmelder: **Karl Storz SE & Co. KG**
**78532 Tuttlingen (DE)**

(72) Erfinder: **Tischler, Jan-Heiko**
**78532 Tuttlingen (DE)**

(54) **BELEUCHTUNGSVORRICHTUNG FÜR EIN MEDIZINISCHES BILDGEBUNGSGERÄT WIE EIN ENDOSKOP, EXOSKOP UND/ODER MIKROSKOP**

(57)     Beleuchtungsvorrichtung (10), insbesondere für ein medizinisches Bildgebungsgerät (11) wie ein Endoskop, Exoskop und/oder Mikroskop, umfassend:
- eine Beleuchtungseinheit (14), welche zu einer Bereitstellung einer Beleuchtung mit einem Beleuchtungsspektrum (54) eingerichtet ist,
- eine weitere Beleuchtungseinheit (60), welche zu einer Bereitstellung einer weiteren Beleuchtung mit einem weiteren Beleuchtungsspektrum (56) eingerichtet ist, und
- eine Kombinationseinheit (65), welches dazu eingerichtet ist, zumindest einen Teil der Beleuchtung und zumindest einen Teil der weiteren Beleuchtung zu einer Bildung einer Kombinationsbeleuchtung zusammenzuführen,
wobei die Kombinationseinheit (65) ein Strahlteilerelement (66) aufweist, welches einen für die Beleuchtung im Wesentlichen reflektierenden ersten Bereich (40) und einen für die weitere Beleuchtung im Wesentlichen transmittierenden zweiten Bereich (42) aufweist, welcher neben dem ersten Bereich (40) angeordnet und bevorzugt von dem ersten Bereich (40) zumindest teilweise umgeben ist.

**Fig. 2**

EP 4 706 497 A1

**Beschreibung**

Die vorliegende Erfindung betrifft eine Beleuchtungsvorrichtung für ein medizinisches Bildgebungsgerät wie ein Endoskop, Exoskop und/oder Mikroskop, eine medizinische, insbesondere endoskopische, exoskopische und/oder mikroskopische, Bildgebungsvorrichtung mit einer solchen Beleuchtungsvorrichtung und ein Verfahren zum Betrieb einer Beleuchtungsvorrichtung.

Aus dem Stand der Technik sind Bildgebungsvorrichtungen wie beispielsweise endoskopische oder exoskopische Vorrichtungen bekannt, die Multispektral- oder Hyperspektralbilder erzeugen. Multispektral- oder Hyperspektralbilder weisen neben zwei räumlichen Dimensionen, wie sie etwa ein herkömmliches Bild einer Kamera hat, eine spektrale Dimension auf. Die spektrale Dimension umfasst mehrere Spektralbänder (Wellenlängenbänder). Multispektrale und hyperspektrale Bilder unterscheiden sich im Wesentlichen in der Anzahl an und der Breite von ihren spektralen Bändern.

Es sind einige Bildgebungsvorrichtungen zur Erzeugung solcher Multispektral- oder Hyperspektralbilder, insbesondere im Kontext medizinischer Anwendungen, bekannt. In DE 20 2014 010 558 U1 ist beispielsweise eine Vorrichtung zur Aufnahme eines Hyperspektralbilds eines Untersuchungsgebiets eines Körpers beschrieben. In der Vorrichtung sind ein Eingangsobjektiv zur Erzeugung eines Bilds in einer Bildebene sowie eine schlitzförmige Blende in der Bildebene zur Ausblendung eines schlitzförmigen Bereichs des Bilds angeordnet. Das durch die Blende hindurchtretende Licht wird mittels eines dispersiven Elements aufgefächert und mittels eines Kamerasensors aufgenommen. Dadurch kann von dem Kamerasensor eine Vielzahl von Spektren mit jeweils zugeordneter räumlicher Koordinate entlang der Längsrichtung der schlitzförmigen Blende aufgenommen werden. Die beschriebene Vorrichtung ist weiterhin dazu eingerichtet, in einer von der Längsrichtung der schlitzförmigen Blende verschiedenen Richtung weitere Spektren entlang der Längsrichtung der schlitzförmigen Blende aufzunehmen. Das dieser Offenbarung zugrunde liegende Verfahren zur Erzeugung von Multispektral- oder Hyperspektralbildern ist auch als sogenanntes Pushbroom-Verfahren bekannt.

Neben dem Pushbroom-Verfahren gibt es weitere Verfahren zur Erzeugung von Multispektral- oder Hyperspektralbildern. Beim sogenannten Whiskbroom-Verfahren wird das Untersuchungsgebiet oder auch Objekt punktweise abgefahren und für jeden Punkt ein Spektrum gewonnen. Im Gegensatz dazu, werden bei dem Staring-Verfahren mehrere Bilder mit denselben räumlichen Koordinaten aufgenommen. Dabei werden von Bild zu Bild verschiedene Spektralfilter und/oder Beleuchtungsquellen verwendet, um spektrale Information aufzulösen. Ferner gibt es Verfahren, gemäß denen durch geeignete optische Elemente wie optische Slicer, Linsen und Prismen ein zweidimensionales Mehrfarbenbild in mehrere spektrale Einzelbilder zerlegt wird, die gleichzeitig auf unterschiedlichen Detektoren oder Detektorbereichen erfasst werden. Dies wird bisweilen als Schnappschuss-Ansatz bezeichnet.

Wie in DE 10 2020 105 458 A1 beschrieben, eignen sich multispektrale und hyperspektrale Bildgebungsvorrichtungen insbesondere als endoskopische Bildgebungsvorrichtung. In diesem Zusammenhang ist multispektrale und/oder hyperspektrale Bildgebung ein fundamentales Einsatzfeld beispielsweise zur Diagnostik sowie zur Beurteilung eines Erfolgs beziehungsweise einer Qualität eines Eingriffs.

Daneben wird insbesondere im medizinischen Bildgebungsbereich Weißlichtbildgebung eingesetzt. Beobachtetes Gewebe wird mit Weißlicht beleuchtet, und mittels einer Kamera oder anderer Bilderfassungssensorik werden Bilder des Gewebes erzeugt, die dann einem Benutzer angezeigt werden können.

Ferner wird Fluoreszenzbildgebung eingesetzt, im Speziellen im medizinischen Bildgebungsbereich. Gewebe wird dabei gezielt in einem bestimmten Wellenlängenbereich beleuchtet, um in bestimmte Entitäten wie beispielsweise Gewebebereiche eingebrachte fluoreszierende Farbstoffmoleküle anzuregen. Das daraufhin emittierte Licht mit größerer Wellenlänge kann durch einen geeignet gewählten Filter beobachtet werden, mittels dessen Anregungslicht ausgeblendet werden kann.

Multimodale Bildgebungsvorrichtungen gestatten es, wahlweise Weißlichtbilder und/oder Multispektralbilder und/oder Fluoreszenzbilder und/oder Hyperspektralbilder aufzunehmen. Beispiele für derartige Bildgebungsvorrichtungen sind multimodale Endoskope und multimodale Exoskope. Zur Realisierung unterschiedlicher Modi können Beleuchtungsvorrichtungen erforderlich sein, die in unterschiedlichen Beleuchtungsmodi betreibbar sind, um bedarfsweise Beleuchtungslicht in unterschiedlichen Spektralbereichen zu erzeugen.

Aus US 10,481,095 B2 und US 11,668,922 B2 sind Beleuchtungsvorrichtungen mit mehreren Beleuchtungsquellen bekannt, deren jeweils emittiertes Licht mittels Strahlteilerelementen zusammengeführt werden kann.

Ausgehend vom Stand der Technik liegt der Erfindung insbesondere aber nicht beschränkt darauf die Aufgabe zugrunde, eine zuverlässige und effiziente Beleuchtung eines Untersuchungsbereichs bereitzustellen.

Diese Aufgabe wird erfindungsgemäß gelöst durch eine Beleuchtungsvorrichtung, eine endoskopische, exoskopische und/oder mikroskopische Bildgebungsvorrichtung und ein Verfahren zum Betrieb einer Beleuchtungsvorrichtung, wie sie hierin beschrieben und in den Ansprüchen definiert sind.

Die Erfindung geht aus von einer Beleuchtungsvorrichtung für ein medizinisches Bildgebungsgerät wie ein Endoskop, Exoskop und/oder Mikroskop, umfassend:

- eine Beleuchtungseinheit , welche zu einer Bereitstellung einer Beleuchtung mit einem Beleuchtungsspektrum eingerichtet ist,

- eine weitere Beleuchtungseinheit , welche zu einer Bereitstellung einer weiteren Beleuchtung mit einem weiteren Beleuchtungsspektrum eingerichtet ist, und

- eine Kombinationseinheit, welche dazu eingerichtet ist, zumindest einen Teil der Beleuchtung und zumindest einen Teil der weiteren Beleuchtung zu einer Bildung einer Kombinationsbeleuchtung zusammenzuführen.

In einem Aspekt der Erfindung weist die Kombinationseinheit ein Strahlteilerelement auf, welches einen für die Beleuchtung im Wesentlichen reflektierenden ersten Bereich und einen für die weitere Beleuchtung im Wesentlichen transmittierenden zweiten Bereich aufweist, welcher neben dem ersten Bereich angeordnet und bevorzugt von dem ersten Bereich zumindest teilweise und besonders bevorzugt vollständig umgeben ist.

In einem weiteren Aspekt der Erfindung, welcher insbesondere auch unabhängig von dem zuvor genannten Aspekt betrachtet werden kann, ist die Kombinationseinheit dazu eingerichtet, das Beleuchtungsspektrum und das weitere Beleuchtungsspektrum jeweils im Wesentlichen unverändert zusammenzuführen.

Die Erfindung betrifft ferner eine medizinische Bildgebungsvorrichtung, insbesondere eine endoskopische, exoskopische und/oder mikroskopische medizinische Bildgebungsvorrichtung, mit der zuvor genannten Beleuchtungsvorrichtung.

Die Erfindung geht ferner aus von einem Verfahren zum Betrieb einer Beleuchtungsvorrichtung, insbesondere für ein medizinisches Bildgebungsgerät wie ein Endoskop, Exoskop und/oder Mikroskop, wobei die Beleuchtungsvorrichtung umfasst:

- eine Beleuchtungseinheit, welche zu einer Bereitstellung einer Beleuchtung mit einem Beleuchtungsspektrum eingerichtet ist, und

- eine weitere Beleuchtungseinheit, welche zu einer Bereitstellung einer weiteren Beleuchtung mit einem weiteren Beleuchtungsspektrum eingerichtet ist,

wobei zumindest ein Teil der Beleuchtung und zumindest ein Teil der weiteren Beleuchtung zu einer Bildung einer Kombinationsbeleuchtung zusammengeführt werden.

In einem Aspekt der Erfindung werden der Teil der Beleuchtung und der Teil der weiteren Beleuchtung zur Bildung der Kombinationsbeleuchtung mittels eines Strahlteilerelements zusammengeführt, welches einen für die Beleuchtung im Wesentlichen reflektierenden ersten Bereich und einen für die weitere Beleuchtung im Wesentlichen transmittierenden zweiten Bereich aufweist, welcher neben dem ersten Bereich angeordnet und bevorzugt von dem ersten Bereich zumindest teilweise und besonders bevorzugt vollständig umgeben ist.

In einem weiteren Aspekt der Erfindung, welcher insbesondere auch unabhängig von dem zuvor genannten Aspekt betrachtet werden kann, werden das Beleuchtungsspektrum und das weitere Beleuchtungsspektrum jeweils im Wesentlichen unverändert zusammengeführt.

Durch die erfindungsgemäßen Merkmale kann eine zuverlässige und effiziente Beleuchtung eines Untersuchungsbereichs bereitgestellt werden, vor allem wenn in bestimmten Wellenlängenbereichen eine Strahlungsleistung gesteigert werden soll, beispielsweise zur Fluoreszenzanregung. Insbesondere kann eine spektrale Veränderung, das heißt insbesondere eine spektrale Zerstückelung, wie sie beispielsweise bei Verwendung üblicher Strahlteiler mit dichroitischen Filtern auftreten kann, vorteilhaft auch bei sich überlappenden Beleuchtungsspektren vermieden werden. Dies kann zu einem natürlicheren Farbeindruck eines abgebildeten Untersuchungsbereichs führen. Zudem können Strahlungsverluste und/oder Streulicht bei der Zusammenführung minimiert werden. Gegenüber dichroitischen Filtern können Kosten und/oder Toleranzanforderungen, insbesondere bei Verwendung von Laserlicht, vorteilhaft reduziert werden. Es kann insbesondere ein vorteilhafter Betrieb in unterschiedlichen Modi ermöglicht werden. Dabei kann ein hoher Grad an Effizienz und/oder Bediensicherheit und Einfachheit erzielt werden. Durch die vorgeschlagene Kombination von Bildgebungsmodi beziehungsweise hierfür verwendeter Beleuchtungsquellen kann die Komplexität von Lichtquelle und/oder Bilderfassung reduziert werden. Es kann eine geringe Anzahl verbauter Beleuchtungsquellen, Filter und/oder zugeordneter optischer Elemente bei zugleich großem Funktionsumfang erreicht werden. Ferner kann Bauraum eingespart werden, wodurch ein hoher Grad an Kompaktheit erzielt werden kann.

Bei dem ersten Bereich und dem zweiten Bereich handelt es sich um räumliche Bereiche des Strahlteilerelements der Kombinationseinheit. Die beiden räumlichen Bereiche unterscheiden sich dabei strukturell voneinander. Beispielsweise kann der erste Bereich ein Material umfassen oder von einem Material oder einer Materialzusammensetzung gebildet sein, welches sich von einem Material oder einer Materialzusammensetzung des zweiten Bereichs unterscheidet. Zudem könnte der zweite Bereich zumindest teilweise und bevorzugt vollständig durch eine Materialausnehmung gebildet sein. Der erste Bereich kann für die weitere Beleuchtung im Wesentlichen undurchlässig, besonders bevorzugt im Wesentlichen reflektierend, sein. Der zweite Bereich kann für die Beleuchtung im Wesentlichen transmittierend sein.

Unter "im Wesentlichen" undurchlässig, reflektierend oder transmittierend kann verstanden werden, dass zumindest 90 %, bevorzugt zumindest 95 % und besonders bevorzugt zumindest 98 % einer Strahlungsintensität nicht durchgelassen, reflektiert oder transmittiert werden.

Darunter, dass die Kombinationseinheit dazu eingerichtet ist, das Beleuchtungsspektrum und das weitere Beleuchtungsspektrum jeweils "im Wesentlichen unverändert" zusammenzuführen, kann vorliegend verstanden werden, dass durch die Kombinationseinheit zur Bildung der Kombinationsbeleuchtung eine Intensität für alle Wellenlängen des Beleuchtungsspektrums entweder beibehalten oder für alle Wellenlängen mit demselben Proportionalitätsfaktor proportional reduziert wird, wobei Entsprechendes für das weitere Beleuchtungsspektrum gelten kann. Insbesondere findet durch die Kombinationseinheit keine Zerstückelung des Beleuchtungsspektrums und des weiteren Beleuchtungsspektrums statt. Es werden insbesondere keine Wellenlängenbereiche des Beleuchtungsspektrums und/oder des weiteren Beleuchtungsspektrums herausgefiltert.

Die Beleuchtungsvorrichtung kann Teil, insbesondere eine Unterbaugruppe, einer medizinischen, insbesondere endoskopischen, exoskopischen und/oder mikroskopischen, Bildgebungsvorrichtung sein. Die Bildgebungsvorrichtung kann ein medizinische Bildgebungsgerät wie ein Endoskop, Exoskop und/oder Mikroskop aufweisen, welches über einen Lichtleiter, der ebenfalls Teil der Bildgebungsvorrichtung sein kann, mit einer Ausgangsbeleuchtung versorgbar ist. Dabei kann die Ausgangsbeleuchtung zumindest teilweise, vorzugsweise zu einem Großteil und besonders bevorzugt im Wesentlichen durch die Kombinationsbeleuchtung gebildet sein. Das Bildgebungsgerät kann dazu eingerichtet sein, zumindest einen Teil, vorzugsweise zumindest einen Großteil und besonders bevorzugt im Wesentlichen die Gesamtheit der Ausgangsbeleuchtung und/oder Kombinationsbeleuchtung zur Ausleuchtung und/oder Beleuchtung eines Untersuchungsbereichs bereitzustellen. Die Bildgebungsvorrichtung und insbesondere die Beleuchtungsvorrichtung kann dazu eingerichtet sein, Licht in einem Wellenlängenbereich von 100 nm bis 1 mm, vorzugsweise von 200 nm bis 1000 nm und besonders bevorzugt von 400 nm bis 1000 nm in Form eines breiten, kontinuierlichen Spektrums und/oder in Form mehrerer, voneinander getrennter Teilspektren bereitzustellen.

Die Bildgebungsvorrichtung und insbesondere das Bildgebungsgerät ist in einigen Ausführungsformen dazu eingerichtet, zur Begutachtung und/oder Beobachtung in einen Hohlraum einführbar zu sein, beispielsweise in eine künstliche und/oder natürliche Kavität, etwa in ein Inneres eines Körpers, in ein Körperorgan, in Gewebe oder dergleichen. Die Bildgebungsvorrichtung und insbesondere das Bildgebungsgerät kann auch dazu eingerichtet sein, zur Begutachtung und/der Beobachtung in ein Gehäuse, eine Verschalung, einen Schacht, ein Rohr oder eine andere, insbesondere künstliche, Struktur einführbar zu sein.

Die Bildgebungsvorrichtung und insbesondere das Bildgebungsgerät kann dazu eingerichtet sein, Gewebeparameter, Bilder von Wunden, Bilder von Körperteilen etc. aufzunehmen. Beispielsweise kann die Bildgebungsvorrichtung dazu eingerichtet sein, ein Operationsfeld abzubilden. Die Bildgebungsvorrichtung und/oder das Bildgebungsgerät kann eine räumlich und spektral auflösende Bilderfassungseinheit umfassen, die zumindest eine Optik und zumindest eine mit der Optik gekoppelte Bilderfassungssensorik umfasst, die dazu eingerichtet sind, eine Bilderfassung eines Bildbereichs durchzuführen, bei der räumlich und spektral aufgelöste Bilddaten erzeugt werden, die sowohl räumliche als auch spektrale Information umfassen.

Die Bilderfassungseinheit und insbesondere die Optik und/oder die Bilderfassungssensorik kann/können zur multispektralen und/oder hyperspektralen Bildgebung eingerichtet sein, im Speziellen dazu, multispektrale und/oder hyperspektrale Bilddaten zu erfassen und/oder zu erzeugen. Multispektrale Bildgebung beziehungsweise multispektrale Bilddaten kann sich dabei insbesondere auf solche Bildgebung beziehen, bei der wenigstens zwei, insbesondere wenigstens drei, und in einigen Fällen wenigstens fünf Spektralbänder voneinander unabhängig erfassbar sind und/oder erfasst werden. Hyperspektrale Bildgebung beziehungsweise hyperspektrale Bilddaten kann sich dabei insbesondere auf solche Bildgebung beziehen, bei der wenigstens 20, wenigstens 50 oder sogar wenigstens 100 Spektralbänder voneinander unabhängig erfassbar sind und/oder erfasst werden.

In einigen Ausführungsformen umfasst die Bildgebungsvorrichtung und/oder das Bildgebungsgerät eine Weißlichtkamera und/oder Sensorik zur Weißlichtbilderfassung. Die Bildgebungsvorrichtung und/oder das Bildgebungsgerät kann zusätzlich zur spektral aufgelösten Bildgebung zur Weißlichtbildgebung eingerichtet sein. Hierfür kann eine separate Optik und/oder eine gemeinsame Optik verwendet werden. Die Weißlichtbildgebung und die spektral aufgelöste Bildgebung können gleichzeitig oder abwechselnd oder zeitweise gleichzeitig und zeitweise nacheinander durchgeführt werden.

In einigen Ausführungsformen umfasst die Bildgebungsvorrichtung und/oder das Bildgebungsgerät Sensorik zur Fluoreszenzbildgebung. Die Bildgebungsvorrichtung und/oder das Bildgebungsgerät kann zusätzlich zur spektral aufgelösten Bildgebung und gegebenenfalls zusätzlich zur Weißlichtbildgebung zur Fluoreszenzbildgebung eingerichtet sein. Hierfür kann eine separate Optik und/oder eine gemeinsame Optik verwendet werden. Die Fluoreszenzbildgebung, gegebenenfalls die Weißlichtbildgebung und die spektral aufgelöste Bildgebung können gleichzeitig oder abwechselnd oder zeitweise gleichzeitig und zeitweise nacheinander durchgeführt werden.

Für einige Anwendungen kann es vorteilhaft sein, eine große spektrale Auflösung verwenden zu können. Es bietet sich dann eine hyperspektrale Bildgebung an. Diese kann mit einer Weißlichtbildgebung und/oder einer Fluoreszenzbildgebung kombiniert sein. Hierdurch ist eine Beobachtung in Echtzeit über ein Weißlichtbild und/oder ein Fluoreszenzbild möglich, auch wenn die Erfassung spektral aufgelöster Bilddaten nur im Wesentlichen in Echtzeit erfolgt, also beispielsweise mehrere Sekunden zur Erstellung eines spektral aufgelösten Bildes benötigt werden.

Für einige Anwendungen kann es vorteilhaft sein, spektrale Bilddaten in Echtzeit zu erzeugen. Dies beinhaltet beispielsweise die Erzeugung eines spektral aufgelösten Bildes in weniger als einer Sekunde oder sogar mehrmals pro Sekunde. Hierbei kann es zweckmäßig sein, auf multispektrale Bildgebung zurückzugreifen. Einer gegebenenfalls geringeren spektralen Auflösung steht dann eine höhere Bildwiederholrate gegenüber. Je nach Anwendung kann es hinreichend sein, nur wenige verschiedene Spektralbereiche und/oder Wellenlängen zu berücksichtigen, beispielsweise zwei oder drei oder vier oder generell weniger als zehn. Hierbei kann wahlweise auf eine zusätzliche Weißlichtbildgebung verzichtet werden. Spektral aufgelöste Bilddaten, die in Echtzeit gewonnen werden, beziehungsweise mehrere Bilder pro Sekunde liefern, können auch zu Überwachungszwecken eingesetzt werden, wobei nicht zwingend ein wiederzugebendes Bild für einen Benutzer erstellt werden muss, sondern die Bilddaten auch im Hintergrund verarbeitet werden können.

Die optische Schnittstelle kann fest oder wahlweise lösbar und verbindbar sein. Zudem kann die optische Schnittstelle mit einer mechanischen Schnittstelle kombiniert sein, sodass eine optische Verbindung beispielsweise automatisch dann hergestellt wird, wenn das Bildgebungsgerät mechanisch angekoppelt wird.

Die Bildgebungsvorrichtung kann eine Steuerung aufweisen, die dazu eingerichtet ist, einen Betriebszustand des Bildgebungsgeräts und einen Beleuchtungsmodus der Beleuchtungsvorrichtung, insbesondere der Beleuchtungseinheit, automatisch aufeinander abzustimmen. Die Steuerung kann dazu eingerichtet sein, die Beleuchtungsvorrichtung und/oder das Bildgebungsgerät anzusteuern, beispielsweise für MSI- oder PDD-Anwendungen.

Die Beleuchtungseinheit kann eine oder mehrere Beleuchtungsquellen aufweisen, beispielsweise eine erste Beleuchtungsquelle und eine zweite Beleuchtungsquelle und insbesondere auch eine dritte Beleuchtungsquelle. Die Beleuchtungseinheit ist zur Bereitstellung von optischer Strahlung, insbesondere im Wellenlängenbereich von 100 nm bis 1 mm, vorzugsweise im Wellenlängenbereich von 200 nm bis 1000 nm, eingerichtet und kann Infrarot-Strahlung, sichtbares Licht und/oder UV-Strahlung umfassen. Durch die optische Strahlung kann ein Untersuchungsbereich ausgeleuchtet werden, beispielsweise mit Weißlicht, und/oder es kann Anregungsstrahlung zur Anregung eines Lumineszenzfarbstoffs, vorzugsweise eines Fluoreszenzfarbstoffs, wie beispielsweise Cyanin 5.5 (Cy 5.5), Indocyaningrün (ICG), Pafolacianin (OTL 38), VisBlue, ViRed, NIR1 oder Fluoreszin, bereitgestellt werden.

Einzelne Beleuchtungsquellen der Beleuchtungseinheit können zur Emission von optischer Strahlung eines bestimmten Wellenlängenbereichs und/oder eines bestimmten Strahlungsspektrums ausgebildet sein. Die Beleuchtungsquellen sind bevorzugt alle zur Emission von optischer Strahlung in unterschiedlichen Wellenlängenbereichen und/oder mit unterschiedlichen Strahlungsspektren ausgebildet, wobei Überlappungen der Strahlungsspektren denkbar wären. So könnte beispielsweise zumindest eine der Beleuchtungsquellen zur Emission von rotem Licht, insbesondere im Wellenlängenbereich von 640 nm bis 780 nm, zumindest eine der Beleuchtungsquellen zur Emission von grünem Licht, insbesondere im Wellenlängenbereich von 490 nm bis 570 nm, und/oder zumindest eine der Beleuchtungsquellen zur Emission von blauem Licht, insbesondere im Wellenlängenbereich 430 nm bis 490 nm, ausgebildet sein. Ferner könnte zumindest eine der Beleuchtungsquellen zur Emission von Infrarot-Strahlung, insbesondere im Wellenlängenbereich von 780 nm bis 1 mm, und/oder zumindest eine der Beleuchtungsquellen zur Emission von UV-Strahlung, insbesondere im Wellenlängenbereich von 100 nm bis 380 nm, ausgebildet sein. Ferner könnte eine der Beleuchtungsquellen zur Emission von sichtbarem Licht, insbesondere im Wellenlängenbereich von 380 bis 780 nm ausgebildet sein.

Die Beleuchtungsquellen können als beliebige, dem Fachmann als sinnvoll erscheinende Lichtemitter ausgebildet sein, beispielsweise als Gasentladungslampen, insbesondere Xenon-Gasentladungslampen. In einer bevorzugten Ausgestaltung umfasst zumindest eine Beleuchtungsquelle der Beleuchtungseinheit eine LED oder ist als eine solche ausgebildet. Besonders bevorzugt umfassen sämtliche Beleuchtungsquellen der Beleuchtungseinheit jeweils eine LED oder sind als solche ausgebildet. Hierdurch kann eine vorteilhafte Beleuchtung bereitgestellt werden, welche insbesondere kostengünstig und/oder elektronisch einfach anzusteuern ist.

Bevorzugt ist die erste Beleuchtungsquelle zur Emission eines kontinuierlichen LED-Lichtspektrums mit einem Intensitätsmaximum bei 940 nm eingerichtet. Bevorzugt ist die zweite Beleuchtungsquelle zur Emission eines kontinuierlichen LED-Lichtspektrums mit einem Intensitätsmaximum bei 660 nm eingerichtet. Bevorzugt ist die dritte Beleuchtungsquelle zur Emission eines kontinuierlichen LED-Lichtspektrums mit Intensitätsmaxima bei 440 nm und 550 nm eingerichtet.

Zudem kann die Beleuchtungseinheit zumindest ein optisches Element zur Strahlformung, zur Strahlteilung und/oder -zusammenführung und/oder zur Strahlumlenkung aufweisen. Das optische Element kann dazu eingerichtet sein, eine von einer oder mehreren der Beleuchtungsquellen bereitgestellte Strahlung zu formen, insbesondere einen optischen Strahl zu bündeln, und/oder ein von einer oder mehreren der Beleuchtungsquellen bereitgestellte Strahlung in zwei Strahlengänge aufzuteilen oder zwei von zumindest zwei der Beleuchtungsquellen bereitgestellte Strahlen zusammenzuführen und/oder einen von einer oder mehreren der Beleuchtungsquellen bereitgestellte Strahlung umzulenken, insbesondere um dadurch die Beleuchtung der Beleuchtungseinheit bereitzustellen.

Die weitere Beleuchtungseinheit kann eine oder mehrere weitere Beleuchtungsquellen aufweisen, beispielsweise eine weitere erste Beleuchtungsquelle und eine weitere zweite Beleuchtungsquelle und insbesondere auch eine weitere dritte Beleuchtungsquelle. Die weitere Beleuchtungseinheit ist zur Bereitstellung von weiterer optischer Strahlung, insbesondere im Wellenlängenbereich von 100 nm bis 1 mm, vorzugsweise im Wellenlängenbereich von 400 nm bis 800 nm, eingerichtet und kann Infrarot-Strahlung, sichtbares Licht und UV-Strahlung umfassen. Durch die weitere optische Strahlung kann ein Untersuchungsbereich ausgeleuchtet werden, bevorzugt mit Weißlicht, und/oder es kann Anregungsstrahlung zur Anregung eines Lumineszenzfarbstoffs, vorzugsweise eine Fluoreszenzfarbstoffs, wie beispielsweise Cyanin 5.5 (Cy 5.5), Indocyaningrün (ICG), Pafolacianin (OTL 38), VisBlue, ViRed, NIR1 oder Fluoreszin, bereitgestellt werden.

Einzelne weitere Beleuchtungsquellen der weiteren Beleuchtungseinheit können zur Emission von weiterer optischer Strahlung eines bestimmten Wellenlängenbereichs und/oder eines bestimmten Strahlungsspektrums ausgebildet sein. Die weiteren Beleuchtungsquellen sind bevorzugt alle zur Emission von weiterer optischer Strahlung in unterschiedlichen Wellenlängenbereichen und/oder mit unterschiedlichen Strahlungsspektren ausgebildet, wobei Überlappungen denkbar sind. So könnte beispielsweise zumindest eine der weiteren Beleuchtungsquellen zur Emission von rotem Licht, insbesondere im Wellenlängenbereich von 640 nm bis 780 nm, zumindest eine der weiteren Beleuchtungsquellen zur Emission von grünem Licht, insbesondere im Wellenlängenbereich von 490 nm bis 570 nm, und/oder zumindest eine der weiteren Beleuchtungsquellen zur Emission von blauem Licht, insbesondere im Wellenlängenbereich 430 nm bis 490 nm, ausgebildet sein. Ferner könnte zumindest eine der weiteren Beleuchtungsquellen zur Emission von Infrarot-Strahlung, insbesondere im Wellenlängenbereich von 780 nm bis 1 mm, und/oder zumindest eine der weiteren Beleuchtungsquellen zur Emission von UV-Strahlung, insbesondere im Wellenlängenbereich von 100 nm bis 380 nm, ausgebildet sein.

Die Beleuchtungsquellen können als beliebige, dem Fachmann als sinnvoll erscheinende Lichtemitter ausgebildet sein, beispielsweise als Gasentladungslampen oder als LEDs. In einer bevorzugten Ausgestaltung umfasst zumindest eine weitere Beleuchtungsquelle der weiteren Beleuchtungseinheit einen Laser oder eine Laserdiode oder ist als ein solcher oder als eine solche ausgebildet. Besonders bevorzugt umfassen sämtliche weiteren Beleuchtungsquellen der weiteren Beleuchtungseinheit jeweils einen Laser oder eine Laserdiode oder sind als solche ausgebildet. Hierdurch kann eine vorteilhaft schmalbandige Beleuchtung bereitgestellt werden. Ferner kann eine Strahlungsleistungsdichte erreicht werden.

Bevorzugt ist die weitere erste Beleuchtungsquelle zur Emission eines Gauß-Strahls und/oder einer Laserlinie mit einer Wellenlänge von 470 nm eingerichtet. Bevorzugt ist die weitere zweite Beleuchtungsquelle zur Emission eines Gauß-Strahls und/oder einer Laserlinie mit einer Wellenlänge von 640 nm eingerichtet. Bevorzugt ist die weitere dritte Beleuchtungsquelle zur Emission eines Gauß-Strahls und/oder einer Laserlinie mit einer Wellenlänge von 770 nm eingerichtet. Ferner wäre denkbar, dass die weitere Beleuchtungseinheit nur eine weitere Beleuchtungsquelle aufweist und diese zur Emission eines Gauß-Strahls und/oder einer Laserlinie mit einer Wellenlänge von 470 nm, 640 nm oder 770 nm eingerichtet ist. Die genannten weiteren Beleuchtungsquellen können jedoch auch zur Emission anderer Wellenlängen eingerichtet sein.

Die weitere Beleuchtungseinheit kann zumindest ein weiteres optisches Element zur Strahlformung, zur Strahlteilung und/oder -zusammenführung und/oder zur Strahlumlenkung aufweisen. Das weitere optische Element kann dazu eingerichtet sein, eine von einer oder mehreren der weiteren Beleuchtungsquellen bereitgestellte Strahlung zu formen, insbesondere einen optischen Strahl zu bündeln, und/oder ein von einer oder mehreren der weiteren Beleuchtungsquellen bereitgestellte Strahlung in zwei Strahlengänge aufzuteilen und/oder zwei von zumindest zwei der weiteren Beleuchtungsquellen bereitgestellte Strahlen zusammenzuführen und/oder einen von einer oder mehreren der weiteren Beleuchtungsquellen bereitgestellte Strahlung umzulenken, insbesondere um dadurch die weitere Beleuchtung der weiteren Beleuchtungseinheit bereitzustellen.

Die Beleuchtungsquellen und/oder die weiteren Beleuchtungsquellen können unabhängig voneinander oder auch in Gruppen und insbesondere auch nur zeitweise und/oder sequentiell aktivierbar sein. Die Beleuchtungseinheit und/oder die weitere Beleuchtungseinheit kann in zumindest einem Multispektralmodus betreibbar sein, in dem eine erste Gruppe der Beleuchtungsquellen und/oder der weiteren Beleuchtungsquellen zumindest zeitweise aktiviert ist und in dem die Beleuchtungseinheit und/oder die weitere Beleuchtungseinheit Beleuchtungslicht für Multispektralbildgebung liefert. Ferner kann die Beleuchtungseinheit und/oder die weitere Beleuchtungseinheit in zumindest einem Fluoreszenzmodus betreibbar sein, in dem eine zweite Gruppe der Beleuchtungsquellen und/oder der weiteren Beleuchtungsquellen zumindest zeitweise aktiviert ist und in dem die Beleuchtungseinheit und/oder die weitere Beleuchtungseinheit Beleuchtungslicht für Fluoreszenzbildgebung liefert. Die Beleuchtungsquellen und/oder die weiteren Beleuchtungsquellen können zumindest eine Beleuchtungsquelle umfassen, das sowohl in der ersten Gruppe als auch in der zweiten Gruppe enthalten ist. Es versteht sich, dass auch gemischte Betriebsmodi vorkommen können, in denen die genannten Modi sequentiell verwendet werden können. Beispielsweise kann sequentiell Multispektralbildgebung und Fluoreszenzbildgebung durchgeführt werden.

In machen Ausgestaltungen überlappt das Beleuchtungsspektrum im Wellenlängenraum zumindest teilweise mit dem weiteren Beleuchtungsspektrum. Hierdurch kann eine Strahlungsleistung im Überlappungsbereich des Wellenlängenraums vorteilhaft angepasst und insbesondere gesteigert werden.

Dabei kann das Beleuchtungsspektrum auch durch einzelne, insbesondere im Wellenlängenraum voneinander getrennte, Beleuchtungseinzelspektren gebildet sein, welche insbesondere von einzelnen Beleuchtungsquellen der Beleuchtungseinheit stammen können. Eines dieser Beleuchtungseinzelspektren kann mit dem weiteren Beleuchtungsspektrum überlappen, welches wiederum durch einzelne, insbesondere im Wellenlängenraum voneinander getrennte, weitere Beleuchtungseinzelspektren gebildet sein kann, welche von weiteren Beleuchtungsquellen der weiteren Beleuchtungseinheit stammen können.

Das Beleuchtungsspektrum kann breitbandiger ausgebildet sein als das weitere Beleuchtungsspektrum, wodurch vorteilhafte Anwendungsmöglichkeiten eröffnet werden können. Insbesondere kann das Beleuchtungsspektrum zur Ausleuchtung des Untersuchungsbereichs herangezogen werden. Das weitere Beleuchtungsspektrum kann hingegen vorteilhaft eine Fluoreszenzanregung unterstützen und/oder ermöglichen.

Hierbei kann sich "breitbandiger" auch darauf beziehen, dass durch Beleuchtungsquellen der Beleuchtungseinheit erzeugte Beleuchtungseinzelspektren breitbandiger sind als durch weitere Beleuchtungsquellen der weiteren Beleuchtungseinheit erzeugte weitere Beleuchtungseinzelspektren.

Die Beleuchtungsvorrichtung kann einen Beleuchtungsausgang umfassen, welcher eine optische Schnittstelle, insbesondere zu einer Verbindung mit dem Lichtleiter, aufweisen und zu einer Bereitstellung der Ausgangsbeleuchtung eingerichtet sein kann.

Die Beleuchtungsvorrichtung kann einen ersten optischen Pfad von der ersten Beleuchtungsquelle zum Beleuchtungsausgang und zumindest einen zweiten optischen Pfad von der zweiten Beleuchtungsquelle zum Beleuchtungsausgang umfassen.

Unter einem "optischen Pfad" kann vorliegend eine Einheit verstanden werden, welche durch optische Elemente, beispielsweise zur Strahlformung, zur Strahlteilung und/oder -zusammenführung und/oder zur Strahlumlenkung, und optische Weglängen entlang eines Strahlengangs definiert ist und welche sich entlang des Strahlengangs zwischen einer der Beleuchtungsquellen und dem Beleuchtungsausgang erstreckt. Die optischen Elemente können dabei Teil des entsprechenden optischen Pfads sein. Der erste optische Pfad und der zweite optische Pfad können sich teilweise überlappen, sodass ein optisches Element des ersten optischen Pfads auch Teil des zweiten optischen Pfads sein kann.

Dabei können der erste optische Pfad und der zweite optische Pfad jeweils genau drei optische Strahlformerelemente, vorzugsweise Linsen, aufweisen.

Unter einem "optischen Strahlformerelement" kann ein optisches Element verstanden werden, welches zur Strahlformung, insbesondere zu einer Strahlbündelung oder Strahlaufweitung, eingerichtet ist. Als optische Strahlformerelemente kommen beispielsweise diffraktive optische Elemente, Achromaten und/oder Einzellinsen, besonders bevorzugt Sammellinsen, in Frage. Dabei sind im Falle optischer Strahlformerelemente, die auf einem Zusammenwirken mehrerer optischer Teilelemente beruhen, beispielsweise mehrerer Linsen eines Achromats, diese Teilelemente nicht einzeln als optische Strahlformerelemente zu zählen, sondern lediglich gemeinsam als ein einzelnes optisches Strahlformerelement. Strahlteilerelemente sind nicht als optische Strahlformerelemente zu verstehen.

Besonders bevorzugt sind die genau drei optischen Strahlformerelemente als eine Kondensorlinse oder ein Kondensor, eine Kompensatorlinse, insbesondere eine Sammellinse, und als eine Fokuslinse ausgebildet. Kondensorlinsen oder Kondensoren können allgemein dazu eingerichtet sein, eine Divergenz einer jeweils zugeordneten Beleuchtungsquelle zu reduzieren und/oder einen möglichst großen Teil einer von der zugeordneten Beleuchtungsquellen bereitgestellten Strahlung in einen abbildenden Strahlengang einzubringen und/oder die bereitgestellte Strahlung zumindest teilweise zu kollimieren. Kondensorlinsen oder Kondensoren können allgemein für eine gleichmäßige Ausleuchtung sorgen. Kompensatorlinsen können allgemein dazu eingerichtet sein, einen sich aufweitenden Strahlengang zu kompensieren und wieder zu verengen, insbesondere zu kollimieren oder zu fokussieren. Dabei sind gegebenenfalls mehrere in einer Kondensorlinse oder einem Kondensor zusammenwirkende Linsen, insbesondere Sammellinsen, nicht einzeln zu zählen, sondern der Verbund dieser Linsen stellt in Form einer Kondensorlinse oder eines Kondensors ein einzelnes optisches Strahlformerelement dar. Entsprechendes gilt für eine Kompensatorlinse.

In manchen Ausführungsformen können sämtliche optischen Pfade von Beleuchtungsquellen der Beleuchtungseinheit zum Beleuchtungsausgang jeweils höchstens und bevorzugt genau drei optische Strahlformerelemente, vorzugsweise Linsen, aufweisen. Hierdurch kann eine geringe Anzahl verbauter Elemente bei zugleich großem Funktionsumfang erreicht werden. Ferner kann Bauraum eingespart werden, wodurch ein hoher Grad an Kompaktheit erzielt werden kann. Durch die Verwendung von maximal drei optischen Strahlformerelementen in den optischen Pfaden kann besonders vorteilhaft eine Angleichung von Abbildungseigenschaften unterschiedlicher Beleuchtungsquellen ermöglicht werden.

In einer weiteren Ausgestaltung kann der erste optische Pfad und/oder der zweite optische Pfad zumindest zwei optisch unmittelbar hintereinander angeordnete Strahlteilerelemente aufweisen, wodurch eine optische Konstruktion vorteilhaft vereinfacht werden kann.

Insbesondere kann eine Angleichung von Abbildungseigenschaften bezüglich unterschiedlicher Beleuchtungsquellen vereinfacht werden. Ein Strahlteilerelement kann in manchen Ausgestaltungen als Kreuzstrahlteiler ausgestaltet sein, welcher zwei winklig, vorzugsweise senkrecht, zueinander angeordnete Einzelstrahlteilern umfasst. Eines der oder beide Strahlteilerelemente kann oder können dazu eingerichtet sein, zumindest zwei Strahlengänge zu vereinen und vorzugsweise in Richtung des Beleuchtungsausgangs zu lenken. Dabei kann einer der Strahlen durch das Strahlteilerelement transmitteiert, der andere Strahl an dem Strahlteilerelement reflektiert werden. Transmissions- und Reflektionseigenschaften des Strahlteilerelements können bevorzugt auf Spektren der beiden Strahlen abgestimmt sein, um Strahlungsverluste vorteilhaft zu vermeiden. Die hierin beschriebenen Strahlteilerelemente und Einzelstrahlteiler können als beliebige, dem Fachmann als sinnvoll erscheinende optische Elemente ausgebildet sein, bevorzugt aufweisend zumindest einen halbdurchlässigen Spiegel und/oder zumindest einen Interferenzspiegel oder -filter, bevorzugt zumindest einen dichroitischen Spiegel und/oder Filter.

Die hierin beschriebenen Strahlteilerelemente oder Einzelstrahlteiler können jeweils als ein beliebiges, dem Fachmann als sinnvoll erscheinendes optisches Element ausgebildet sein, bevorzugt jedoch als ein halbdurchlässiger Spiegel und/oder ein Interferenzspiegel oder -filter, bevorzugt als ein dichroitischer Spiegel und/oder Filter. Das Strahlteilerelement kann dazu eingerichtet sein, zwei Strahlengänge zu vereinen und in Richtung des Beleuchtungsausgangs zu lenken. Dabei wird einer der Strahlen durch das Strahlteilerelement transmitteiert, der andere Strahl an dem Strahlteilerelement reflektiert. Transmissions- und Reflektionseigenschaften des Strahlteilerelement können bevorzugt auf Spektren der beiden Strahlen abgestimmt sein, um Strahlenverluste vorteilhaft zu vermeiden.

Die hierin beschriebenen Strahlteilerelemente oder Einzelstrahlteiler können Bandpassfilter, insbesondere Kerbfilter ("notch filter"), umfassen, sodass sie jeweils in einem schmalen Spektralband oder in schmalen Spektralbändern einen hohen Reflektionsgrad und einen geringen Transmissionsgrad aufweisen, ansonsten aber einen hohen Transmissionsgrad und einen geringen Reflektionsgrad, oder umgekehrt. Alternativ oder zusätzlich können die Strahlteilerelemente oder Einzelstrahlteiler Kantenfilter ("edge filter") umfassen, sodass sie in einem Spektralband bis zu einer Kante einen hohen Reflektionsgrad und einen geringen Transmissionsgrad aufweisen, ansonsten aber einen hohen Transmissionsgrad und einen geringen Reflektionsgrad, oder umgekehrt. Die spektrale Position und/oder Breite der entsprechenden Kerbe und/oder die spektrale Position der Kante kann an den Spektralbereich der jeweils zugeordneten Beleuchtungsquelle oder der jeweils zugeordneten Beleuchtungsquellen angepasst sein, sodass dessen oder deren Licht weitgehend umgelenkt, Licht anderer Beleuchtungsquellen aber weitgehend transmittiert werden kann.

Die Strahlteilerelemente können in einem optischen Strahlengang ausgehend von den Beleuchtungsquellen beide vor oder beide nach der Kompensatorlinse angeordnet sei. Zudem wäre denkbar, dass eines der Strahlteilerelemente vor der Kompensatorlinse und das andere Strahlteilerelement nach der Kompensatorlinse angeordnet ist. Insgesamt könnte der erste optische Pfad und/oder der zweite optische Pfad drei Strahlteilerelemente umfassen. Eines der Strahlteilerelemente könnte als das Strahlteilerelement der Kombinationseinheit ausgebildet sein.

Vorteilhaft sind der erste optische Pfad und der zweite optische Pfad gleich lang, wodurch Abweichungen und gegebenenfalls Messfehler vermieden werden können, die auf relative spektrale Intensitäten in unterschiedlichen Spektralbereichen zurückgehen und beispielsweise auftreten können, wenn ein Endoskop(schaft) relativ zu einer Kameraeinheit verdreht wird und/oder wenn ein Lichtleiter relativ zum Bildgebungsgerät verdreht wird. Aufgrund der im Wesentlichen gleich langen Lichtwege können weitgehend identische Intensitätsprofile der betroffenen Beleuchtungsquellen erzielt werden. Unter "gleich lang" soll hier und im Folgenden verstanden werden, dass zwei Längen im Rahmen von Toleranzen, insbesondere im Rahmen von Fertigungs- und Montagetoleranzen, gleich sind und/oder dass eine relative Abweichung der zwei Längen maximal 1 %, vorzugsweise maximal 0,1 % und besonders bevorzugt maximal 0,01 % beträgt.

In einigen Ausgestaltungen können der erste optische Pfad und der zweite optische Pfad die gleichen optischen Strahlformelemente, vorzugsweise Linsen, aufweisen. Hierdurch können für die verschiedenen optischen Pfade identische Abbildungseigenschaften und/oder Lichtintensitäten ermöglicht werden. Dabei können der erste optische Pfad und der zweite optische Pfad auch teilweise dieselben optischen Strahlformerelemente aufweisen, die sowohl Teil des ersten als auch des zweiten optischen Pfads sind. Bevorzugt weisen der erste optische Pfad und der zweite optische Pfad typgleiche Kondensorlinsen oder Kondensoren auf.

In einigen Ausgestaltungen können der erste optische Pfad und der zweite optische Pfad zueinander identisch sein. Unter "identischen" optischen Pfaden können hierbei optische Pfade verstanden werden, welche gleich lang sind und welche die gleichen, möglicherweise teilweise dieselben, insbesondere gemeinsam genutzten, optischen Elemente aufweisen. Hierdurch können für die beiden optischen Pfade identische Abbildungseigenschaften sichergestellt werden, wodurch insbesondere eine Abbildungsqualität besonders vorteilhaft gesteigert werden kann.

In einer Weiterbildung weisen der erste optische Pfad vorzugsweise eine erste Kondensorlinse oder einen ersten Kondensor, der zweite optische Pfad vorzugsweise eine zweite Kondensorlinse oder einen zweiten Kondensor und der erste optische Pfad und der zweite optische Pfad vorzugsweise eine gemeinsame Kompensatorlinse auf. Hierdurch kann eine Konstruktion vorteilhaft vereinfacht werden. Durch eine gemeinsame Verwendung bestimmter optischer Elemente können zudem Toleranzeinflüsse minimiert werden, wodurch besonders vorteilhaft weitgehend gleiche Abbildungseigenschaften für verschiedene Beleuchtungsquellen erreicht werden können. Bevorzugt ist die erste Kondensorlinse typgleich zur zweiten Kondensorlinse oder der erste Kondensor typgleich zum zweiten Kondensor. Der erste optische Pfad und der zweite optische Pfad können eine gemeinsame Fokuslinse aufweisen, wobei die gemeinsame Fokuslinse vor dem Beleuchtungsausgang angeordnet sein kann. In Strahlrichtung betrachtet kann die gemeinsame Kompensatorlinse zwischen der erste Kondensorlinse oder dem ersten Kondensor beziehungsweise der zweiten Kondensorlinse oder dem zweiten Kondensor und der gemeinsamen Fokuslinse angeordnet sein.

In einer bevorzugten Ausgestaltung kann die Beleuchtungsvorrichtung einen dritten optischen Pfad von einer dritten Beleuchtungsquelle der Beleuchtungseinheit zum Beleuchtungsausgang aufweisen, wobei der dritte optische Pfad eine dritte Kondensorlinse oder einen dritten Kondensor und eine dedizierte Kompensatorlinse oder die gemeinsame Kompensatorlinse aufweisen kann. Hierdurch kann eine Flexibilität, insbesondere hinsichtlich einer Bauraumausnutzung, vorteilhaft gesteigert werden. Ferner kann ein Bauraumbedarf gegebenenfalls reduziert werden, insbesondere durch eine entsprechende Umlenkung eines Strahlengangs. Der dritte optische Pfad kann sich teilweise mit dem ersten optischen Pfad und/oder dem zweiten optischen Pfad überlappen, sodass ein optisches Element des dritten optischen Pfads auch Teil des ersten optischen Pfads und/oder des zweiten optischen Pfads sein kann. Die dritte Kondensorlinse kann typgleich oder auch typverschieden zur ersten und/oder zweiten Kondensorlinse sein. Entsprechend kann auch der dritte Kondensor typgleich oder typverschieden zum ersten und/oder zweiten Kondensor sein. Der dritte optische Pfad und der erste optische Pfad und/oder der zweite optische Pfad können eine gemeinsame Fokuslinse, insbesondere die zuvor genannte gemeinsame Fokuslinse aufweisen, wobei die gemeinsame Fokuslinse vor dem Beleuchtungsausgang angeordnet sein kann. In Strahlrichtung betrachtet kann die dedizierte Kompensatorlinse zwischen der dritten Kondensorlinse oder dem dritten Kondensor und der gemeinsamen Fokuslinse angeordnet sein.

In einigen Ausgestaltungen können der erste optische Pfad, der zweite optische Pfad und der dritte optische Pfad und besonders bevorzugt sämtliche optische Pfade von Beleuchtungsquellen der Beleuchtungseinheit zum Beleuchtungsausgang zueinander identisch sein. Hierdurch können für die drei optischen Pfade und besonders bevorzugt für sämtliche optischen Pfade von Beleuchtungsquellen der Beleuchtungseinheit zum Beleuchtungsausgang identische Abbildungseigenschaften sichergestellt werden, wodurch insbesondere eine Abbildungsqualität besonders vorteilhaft gesteigert werden kann.

In manchen Ausgestaltungen können die erste Beleuchtungsquelle, die zweite Beleuchtungsquelle und/oder die dritte Beleuchtungsquelle zu einer gleichzeitigen Beleuchtung, insbesondere Weißlichtbeleuchtung, und/oder gemeinsamen Auswertung eingerichtet sein. Hierdurch kann eine Abbildungs- und Auswertequalität besonders vorteilhaft gesteigert werden. Die Steuerung kann dazu eingerichtet sein, die erste Beleuchtungsquelle, die zweite Beleuchtungsquelle und/oder die dritte Beleuchtungsquelle in zumindest einem Betriebsmodus gleichzeitig zu betreiben und/oder spektrale Signale aus einem Wellenlängenbereich der ersten Beleuchtungsquelle, der zweiten Beleuchtungsquelle und/oder der dritten Beleuchtungsquelle im Rahmen einer Analyse und/oder Berechnung gleichzeitig zu verwenden.

Die Beleuchtungsvorrichtung kann zusätzlich einen weiteren ersten optische Pfad von der weiteren ersten Beleuchtungsquelle zum Beleuchtungsausgang umfassen. Das Strahlteilerelement der Kombinationseinheit kann dabei ein in Strahlrichtung letztes Strahlteilerelement vor dem Beleuchtungsausgang sein.

In manchen Ausgestaltungen kann der weitere erste optische Pfad genau zwei oder genau drei optische Strahlformerelemente, vorzugsweise Linsen, aufweisen, wodurch eine geringe Anzahl verbauter optischer Elemente bei zugleich großem Funktionsumfang erreicht werden.

Ferner kann Bauraum eingespart werden, wodurch ein hoher Grad an Kompaktheit erzielt werden kann. Durch die Verwendung von genau zwei oder genau drei optischen Strahlformerelementen in dem optischen Pfad kann besonders vorteilhaft eine Angleichung von Abbildungseigenschaften unterschiedlicher Beleuchtungsquellen ermöglicht werden. Besonders bevorzugt sind die genau zwei oder genau drei optischen Strahlformerelemente als eine Kondensorlinse oder ein Kondensor und eine Fokuslinse und möglicherweise zusätzlich eine Kompensatorlinse, insbesondere eine Sammellinse, ausgebildet. Die Fokuslinse kann dabei als die zuvor genannte gemeinsame Fokuslinse ausgebildet sein.

In weiteren Ausgestaltungen kann die Beleuchtungsvorrichtung einen weiteren zweiten optischen Pfad von der weiteren zweiten Beleuchtungsquelle zum Beleuchtungsausgang umfassen, wobei der weitere zweite optische Pfad genau zwei oder genau drei optische Strahlformerelemente, vorzugsweise Linsen, aufweisen kann. Hierdurch kann eine geringe Anzahl verbauter optischer Elemente bei zugleich großem Funktionsumfang erreicht werden. Ferner kann Bauraum eingespart werden, wodurch ein hoher Grad an Kompaktheit erzielt werden kann. Durch die Verwendung von genau zwei oder genau drei optischen Strahlformerelementen in dem optischen Pfad kann besonders vorteilhaft eine Angleichung von Abbildungseigenschaften unterschiedlicher Beleuchtungsquellen ermöglicht werden. Besonders bevorzugt sind die genau zwei oder genau drei optischen Strahlformerelemente als eine Kondensorlinse oder ein Kondensor und eine Fokuslinse und möglicherweise zusätzlich eine Kompensatorlinse, insbesondere eine Sammellinse, ausgebildet. Die Fokuslinse kann dabei als die zuvor genannte gemeinsame Fokuslinse ausgebildet sein.

Der weitere erste optische Pfad und der weitere zweite optische Pfad können gleich lang sind, wodurch Abweichungen und gegebenenfalls Messfehler vermieden werden können, die auf relative spektrale Intensitäten in unterschiedlichen Spektralbereichen zurückgehen und beispielsweise auftreten können, wenn ein Endoskop(schaft) relativ zu einer Kameraeinheit verdreht wird und/oder wenn ein Lichtleiter relativ zum Bildgebungsgerät verdreht wird. Aufgrund der im Wesentlichen gleich langen Lichtwege können weitgehend identische Intensitätsprofile der betroffenen weiteren Beleuchtungsquellen erzielt werden.

In manchen Ausgestaltungen können der weitere erste optische Pfad und der weitere zweite optische Pfad die gleichen optischen Strahlformerelemente, vorzugsweise Linsen, aufweisen. Hierdurch können für die verschiedenen weiteren optischen Pfade identische Abbildungseigenschaften ermöglicht werden. Dabei können der weitere erste optische Pfad und der weitere zweite optische Pfad auch teilweise dieselben optischen Strahlformerelemente aufweisen, die sowohl Teil des weiteren ersten als auch des weiteren zweiten optischen Pfads sind. Bevorzugt weisen der weitere erste optische Pfad und der weitere zweite optische Pfad typgleiche Kondensorlinsen oder Kondensoren auf.

In bestimmten Ausgestaltungen kann für sämtliche (weiteren) Beleuchtungsquellen der Beleuchtungseinheit und der weiteren Beleuchtungseinheit ein optischer Pfad von der jeweiligen (weiteren) Beleuchtungsquelle zum Beleuchtungsausgang identisch ist. Hierdurch können für sämtliche optische Pfade der Beleuchtungsvorrichtung identische Abbildungseigenschaften sichergestellt werden, wodurch insbesondere eine Abbildungs- und Beleuchtungsqualität besonders vorteilhaft gesteigert werden kann.

Dabei kann der erste optische Pfad und/oder der zweite optische Pfad und/oder der weitere erste optische Pfad und/oder der weitere zweite optische Pfad zumindest ein Strahlumlenkelement zu einer reinen Umlenkung eines Strahlengangs aufweisen. Hierdurch kann eine Flexibilität hinsichtlich einer Konstruktion, insbesondere bezüglich einer Anordnung von Komponenten, einer Bauraumausnutzung und/oder einer Kühlung, vorteilhaft gesteigert werden. Das Strahlumlenkelement kann dabei als ein beliebiges, dem Fachmann als sinnvoll erscheinendes optisches Element ausgebildet sein, beispielsweise als ein Lichtleiter und/oder bevorzugt als ein Spiegel.

Ferner kann die Beleuchtungsvorrichtung eine Detektoreinheit zu einer Bestimmung zumindest einer Beleuchtungseigenschaft der Ausgangsbeleuchtung und/oder der Kombinationsbeleuchtung umfassen. Hierdurch kann eine Abbildungs-, Beleuchtungs- und/oder Auswertequalität vorteilhaft gesteigert werden. Dabei können insbesondere thermische Einflüsse auf das Beleuchtungsspektrum beziehungsweise die Beleuchtungsspektren durch eine Erwärmung und/oder Alterung von Beleuchtungsquellen und/oder optischen Elementen berücksichtigt und/oder kompensiert werden. Hierdurch kann ein Weißabgleich verbessert und/oder ein anfänglicher Weißabgleich korrigiert werden. Die Detektoreinheit kann dabei zumindest einen beliebigen, dem Fachmann als sinnvoll erscheinenden optischen Detektor aufweisen, beispielsweise eine Fotodiode, ein Fototransistor, einen Fotowiderstand, einen CCD-Sensor, einen CMOS-Sensor und/oder eine Kamera. Die Beleuchtungsvorrichtung kann mit dem Bildgebungsgerät, insbesondere der Bilderfassungseinheit, verbunden sein, wodurch ein Feedback aus dem Bildgebungsgerät der Beleuchtungsvorrichtung bereitgestellt werden kann, insbesondere für den Weißabgleich.

Dabei könnte die Beleuchtungseigenschaft ein Beleuchtungsspektrum, eine Beleuchtungsintensität und/oder eine Beleuchtungsleistung sein. Vorzugsweise ist die Beleuchtungseigenschaft eine Gesamtstrahlungsleistung der Ausgangsbeleuchtung und/oder Kombinationsbeleuchtung, wodurch eine Abbildungs-, Beleuchtungs- und/oder Auswertequalität besonders einfach gesteigert werden kann, wobei auf einfache Weise thermische Einflüsse berücksichtigt und/oder kompensiert werden können. Insbesondere kann ein Weißabgleich besonders einfach verbessert und/oder korrigiert werden.

In manchen Ausgestaltungen kann der zweite Bereich für die Beleuchtung zumindest teilweise und in bestimmten Ausgestaltungen im Wesentlichen transmittierend sein, wodurch eine Detektion und/oder eine Überwachung einer Strahlungsleistung und/oder -zusammensetzung der Beleuchtung und/oder der Kombinationsbeleuchtung und/oder der Ausgangsbeleuchtung vorteilhaft ermöglicht werden kann.

Hierzu kann die Detektoreinheit zum Einsatz kommen, welche zu einem Nachweis eines Anteils der Beleuchtung eingerichtet ist, welcher den zweiten Bereich passiert hat. Insofern kann die Detektoreinheit auf einer der Beleuchtungseinheit abgewandten Seite des Strahlteilerelements der Kombinationseinheit angeordnet sein.

Wenn der zweite Bereich für die weitere Beleuchtung zumindest teilweise reflektierend ist, kann eine Detektion und/oder eine Überwachung einer Strahlungsleistung und/oder -zusammensetzung der weiteren Beleuchtung und/oder der Kombinationsbeleuchtung und/oder der Ausgangsbeleuchtung vorteilhaft ermöglicht werden.

Dabei kann ein Anteil der weiteren Beleuchtung, welcher am zweiten Bereich reflektiert wird, weniger als 10 %, bevorzugt weniger als 5 % und besonderes bevorzugt weniger als 2 % einer Strahlungsintensität der weiteren Beleuchtung darstellen.

Hierzu kann die Detektoreinheit zum Einsatz kommen, welche zu einem Nachweis des Anteils der weiteren Beleuchtung eingerichtet ist, welche am zweiten Bereich reflektiert wurde. Insofern kann die Detektoreinheit auf einer der weiteren Beleuchtungseinheit zugewandten Seite des Strahlteilerelements der Kombinationseinheit angeordnet sein.

In manchen Ausgestaltungen kann das Strahlteilerelement der Kombinationseinheit einen Breitbandspiegel mit integrierender Lochblende umfassen, welche den zweiten Bereich definiert. Hierdurch kann eine vorteilhaft einfache Kombinationseinheit bereitgestellt werden, welche insbesondere das Beleuchtungsspektrum und das weitere Beleuchtungsspektrum jeweils im Wesentlichen unverändert zusammenzuführen kann.

In diesem Fall ist der zweite Bereich durch den Bereich der Lochblende definiert. Die Lochblende kann einen Durchmesser von zumindest 1 mm, insbesondere zumindest 2 mm, und von höchstens 5 mm, insbesondere zumindest 4 mm, haben. Sie kann an einer beliebigen Stelle des Breitbandspiegels angeordnet sein. Der Breitbandspiegel kann eben oder gewölbt, insbesondere parabolisch, ausgebildet sein.

Dabei kann eine Ausrichtung einer Begrenzungswand oder von Begrenzungswänden der Lochblende senkrecht oder winklig zu einer Hauptoberfläche des Strahlteilerelements der Kombinationseinheit, insbesondere des Breitbandspiegels, sein, wodurch insbesondere einstellbar ist, inwieweit die Beleuchtung und/oder die weitere Beleuchtung den zweiten Bereich passieren kann. Somit kann eine Bohrung zur Herstellung der Lochblende senkrecht zur Hauptoberfläche oder unter einem Winkel kleiner 90° zur Hauptoberfläche erfolgen.

Das Strahlteilerelement der Kombinationseinheit kann ein Füllelement umfassen, welches die Lochblende zumindest teilweise und bevorzugt vollständig ausfüllt. Das Füllelement könnte beispielsweise ein transparentes Fensterelement sein. Das Strahlteilerelement der Kombinationseinheit kann im zweiten Bereich ein optisches Filterelement aufweisen, wodurch Transmissions- und Reflektionseigenschaften des zweiten Bereichs vorteilhaft veränderbar und/oder einstellbar sind.

Beispielsweise könnte das optische Filterelement über einen Wellenlängenbereich der Beleuchtung und/oder der weiteren Beleuchtung hinweg teildurchlässig sein und einen geringen Anteil, insbesondere weniger als 10 %, bevorzugt weniger als 5 % und besonders bevorzugt weniger als 2 %, einer auftreffenden Lichtintensität reflektieren und ansonsten das Licht im Wesentlichen transmittieren.

Zudem könnte das optische Filterelement für die Beleuchtung im Wesentlichen reflektierend und zumindest teilweise transmittierend sein, wobei ein Anteil der Beleuchtung, welcher am optischen Filterelement transmittiert wird, weniger als 10 %, bevorzugt weniger als 5 % und besonderes bevorzugt weniger als 2 % einer Strahlungsintensität der Beleuchtung darstellen kann. Ferner könnte das optisches Filterelement für die weitere Beleuchtung im Wesentlichen transmittierend und zumindest teilweise reflektierend sein, wobei ein Anteil der weiteren Beleuchtung, welcher am optischen Filterelement reflektiert wird, weniger als 10 %, bevorzugt weniger als 5 % und besonderes bevorzugt weniger als 2 % einer Strahlungsintensität der weiteren Beleuchtung ausmachen kann. Insbesondere könnte das optische Filterelement für jede weitere Beleuchtungsquelle der weiteren Beleuchtungseinheit eine dedizierte Kerbe ("notch") aufweisen.

In manchen Ausgestaltungen kann das Strahlteilerelement der Kombinationseinheit ein für die Beleuchtung und die weitere Beleuchtung im Wesentlichen transmittierendes Substrat und eine Beschichtung auf dem Substrat aufweisen, welche im ersten Bereich breitbandig reflektiert und welche im zweiten Bereich für die weitere Beleuchtung zumindest teilweise und vorzugsweise im Wesentlichen transmittierend ausgebildet ist. Hierdurch kann eine vorteilhaft einfache Herstellung ermöglicht werden, wodurch insbesondere Kosten reduziert werden können. Ferner kann eine hohe Zuverlässigkeit erreicht werden.

Insbesondere kann die Beschichtung im zweiten Bereich eine Materialausnehmung aufweisen. Alternativ kann die Beschichtung im zweiten Bereich für die Beleuchtung zumindest teilweise und vorzugsweise im Wesentlichen reflektierend ausgebildet sein.

Dabei kann die Beschichtung zweiteilig mit einem ersten Beschichtungsabschnitt im ersten Bereich und einem zweiten Beschichtungsabschnitt im zweiten Bereich ausgebildet sein, wobei der erste Beschichtungsabschnitt bezüglich einer Hauptoberfläche des Substrats neben dem zweiten Beschichtungsabschnitt angeordnet sein kann und diesen zumindest teilweise und bevorzugt vollständig umschließen kann.

Die Beschichtung und insbesondere der erste Beschichtungsabschnitt und/oder der zweite Beschichtungsabschnitt kann dabei mittels beliebiger, dem Fachmann als sinnvoll erscheinender Beschichtungs-, Aufdampf- und/oder Druckverfahren auf das Substrat aufgebracht sein.

Dabei kann die Beschichtung im zweiten Bereich einen optischen Filter bilden, wodurch Transmissions- und Reflektionseigenschaften des zweiten Bereichs vorteilhaft veränderbar und/oder einstellbar sind.

Hinsichtlich der optischen Eigenschaften des optischen Filters sei auf obige Beschreibung zu den optischen Eigenschaften des optischen Filterelements verwiesen, welche hier für den optischen Filter gleichermaßen denkbar sind.

Die Beleuchtungseinheit kann eine Optikeinheit aufweisen, welche dazu eingerichtet ist, die Beleuchtung am Strahlteilerelement der Kombinationseinheit mit einem Strahlquerschnitt bereitzustellen, welcher den zweiten Bereich vollständig und den ersten Bereich zumindest abschnittsweise überspannt. Hierdurch kann eine hohe Ausbeute der Beleuchtung ermöglicht werden.

Eine Fläche des Strahlquerschnitts der Beleuchtung kann dabei am Strahlteilerelement der Kombinationseinheit zumindest 200 mm$^2$, vorzugsweise zumindest 300 mm$^2$ und besonders bevorzugt zumindest 400 mm$^2$ betragen. Die Fläche des Strahlquerschnitts der Beleuchtung kann am Strahlteilerelement der Kombinationseinheit höchstens 2500 mm$^2$, vorzugsweise höchstens 2250 mm$^2$ und besonders bevorzugt höchstens 2000 mm$^2$ betragen.

Die Optikeinheit kann dabei eine Kondensorlinse, insbesondere eine der zuvor genannten Kondensorlinsen, oder einen Kondensor, insbesondere einen der zuvor genannten Kondensoren, eine Kompensatorlinse, insbesondere eine der zuvor genannten Kompensatorlinsen, und/oder eine Fokuslinse, insbesondere die zuvor genannte Fokuslinse, umfassen. Dabei kann die Beleuchtung beim Auftreffen auf das Strahlteilerelement der Kombinationseinheit kollimiert oder im Wesentlichen beziehungsweise ungefähr kollimiert sein.

Wenn dabei eine Fläche des Strahlquerschnitts um zumindest einen Faktor 2, vorzugsweise zumindest einen Faktor 4, bevorzugt zumindest einen Faktor 10 und besonders bevorzugt zumindest einen Faktor 100, größer ist als eine Lichteintrittsfläche des zweiten Bereichs, kann eine besonders vorteilhaft hohe Lichtausbeute der Beleuchtung sichergestellt werden.

In machen Ausgestaltungen kann die weitere Beleuchtungseinheit eine weitere Optikeinheit aufweisen, welche dazu eingerichtet ist, die weitere Beleuchtung so zu fokussieren, dass diese das Strahlteilerelement der Kombinationseinheit lediglich innerhalb des zweiten Bereichs passiert. Hierdurch kann eine Lichtausbeute der weiteren Beleuchtung maximiert werden. Ferner kann eine Erwärmung des Strahlteilerelements der Kombinationseinheit verringert oder gar vermieden werden.

Die Lichteintrittsfläche des zweiten Bereichs kann dabei um einen Faktor 2 bis 3 größer sein als eine Fokusquerschnittsfläche der weiteren Beleuchtung im zweiten Bereich.

Die weitere Optikeinheit kann dabei eine Kondensorlinse, insbesondere eine der zuvor genannten Kondensorlinsen, oder einen Kondensor, insbesondere einen der zuvor genannten Kondensoren, eine Kompensatorlinse, insbesondere eine der zuvor genannten Kompensatorlinsen, und/oder eine Fokuslinse, insbesondere die zuvor genannte Fokuslinse, umfassen.

Die Beleuchtungsvorrichtung kann eine Sammellinse, insbesondere die zuvor genannte Fokuslinse, umfassen, welche dazu eingerichtet ist, die Kombinationsbeleuchtung auf die optische Schnittstelle zu fokussieren. Hierdurch kann eine Lichtausbeute und/oder -homogenität vorteilhaft gesteigert werden.

Die Beleuchtungsvorrichtung kann vor dem Beleuchtungsausgang optional einen Homogenisator zur Homogenisierung der Ausgangsbeleuchtung aufweisen.

Sind Objekte in diesem Dokument mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Entsprechendes gilt für den Bezeichner "weitere", "weiterer" bzw. "weiteres".

Die hierin offenbarten Vorrichtungen, Einheiten und Systeme sollen hierbei nicht auf die oben beschriebene Anwendung und Ausführungsform beschränkt sein. Insbesondere können diese zu einer Erfüllung einer hierin beschriebenen Funktionsweise eine von einer hierin genannten Anzahl von einzelnen Elementen, Bauteilen und Einheiten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist gegebenenfalls nur eines davon in den Figuren und in der Beschreibung mit einem Bezugszeichen versehen. Die Beschreibung dieses Exemplars kann entsprechend auf die anderen Exemplare von dem Objekt übertragen werden.

Es zeigen:

Fig. 1      eine medizinische Bildgebungsvorrichtung mit einem Bildgebungsgerät in Form eines Endoskops und mit einer mit dem Endoskop verbundenen Beleuchtungsvorrichtung,

Fig. 2      eine schematische Darstellung der Beleuchtungsvorrichtung, welche eine Beleuchtungseinheit und eine weitere Beleuchtungseinheit aufweist,

Fig. 3      eine schematische Darstellung eines Strahlengangs der Beleuchtungsvorrichtung, welche eine Kombinationseinheit zur Bildung einer Kombinationsbeleuchtung aus einer Beleuchtung der Beleuchtungseinheit und einer weiteren Beleuchtung der weiteren Beleuchtungseinheit aufweist,

Fig. 4      ein Strahlteilerelement der Kombinationseinheit, welches einen Breitbandspiegel mit integrierender Lochblende umfasst, in einer seitlichen Schnittdarstellung,

Fig. 5      eine Draufsicht auf das Strahlteilerelement mit eingezeichnetem Strahlquerschnitt der Beleuchtung,

Fig. 6    ein Schaubild mit einem Beleuchtungsspektrum der Beleuchtung und einem weiteren Beleuchtungsspektrum der weiteren Beleuchtung,

Fig. 7    ein Schaubild eines Verfahrens zum Betrieb der Beleuchtungsvorrichtung,

Fig. 8    eine Ausführungsvariante eines Strahlteilerelements, welches in einer Lochblende ein optisches Filterelement aufweist, in einer seitlichen Schnittdarstellung und

Fig. 9    ein weiteres Ausführungsbeispiel eines Strahlteilerelements, welches ein transmittierendes Substrat mit einer Beschichtung aufweist, in einer seitlichen Schnittdarstellung.

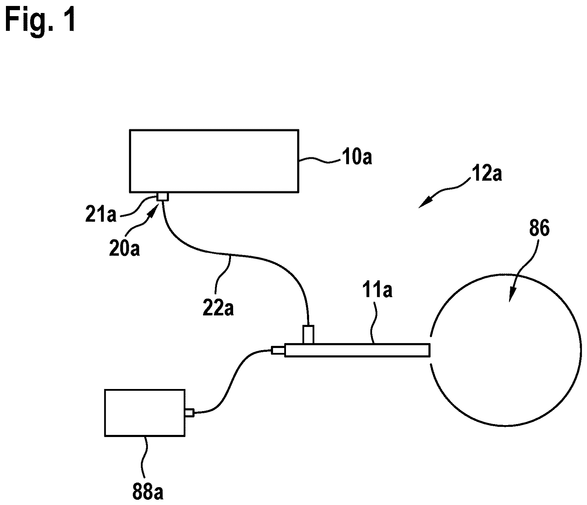

Fig. 1 zeigt eine schematische Darstellung einer medizinischen Bildgebungsvorrichtung 12a. Im exemplarisch dargestellten Fall ist die Bildgebungsvorrichtung 12a eine endoskopische medizinische Bildgebungsvorrichtung. Alternativ könnte es sich bei der medizinischen Bildgebungsvorrichtung 12a um eine exoskopische, eine mikroskopische oder eine makroskopische medizinische Bildgebungsvorrichtung handeln. Die Bildgebungsvorrichtung 12a ist beispielsweise zu einer Untersuchung einer Kavität 86a eingerichtet.

Die Bildgebungsvorrichtung 12a weist ein medizinisches Bildgebungsgerät 11a auf. Im dargestellten Fall handelt es sich hierbei um ein Endoskop, das teilweise in die Kavität 86a einführbar ist.

Die Bildgebungsvorrichtung 12a umfasst eine Beleuchtungsvorrichtung 10a mit einer optischen Schnittstelle 21a. Das Bildgebungsgerät 11a ist optisch an die optische Schnittstelle 21a anbindbar. Die optische Schnittstelle 21a kann Teil einer optisch-mechanischen Schnittstelle sein, die wahlweise lösbar und verbindbar ist. Das Bildgebungsgerät 11a kann wahlweise von der Beleuchtungsvorrichtung 10a abkoppelbar sein.

Die Beleuchtungsvorrichtung 10a ist zur Bereitstellung einer Ausgangsbeleuchtung an einem Beleuchtungsausgang 20a eingerichtet, welcher die optische Schnittstelle 21a aufweist. Die Ausgangsbeleuchtung ist mittels eines Lichtleiters 22a der Bildgebungsvorrichtung 12a dem Bildgebungsgerät 11a zuführbar. Das Bildgebungsgerät 11a koppelt die Ausgangsbeleuchtung auf ein abzubildendes Objekt, wie beispielsweise einen Situs, aus.

Die Bildgebungsvorrichtung 12a umfasst im dargestellten Fall ferner eine Anzeigeeinheit 88a, auf welcher Bilder angezeigt werden können, die auf Bilddaten beruhen, die mittels des Bildgebungsgeräts 11a erfasst wurden. Hierbei kann es sich um Videobilder, Standbilder, Überlagerungen unterschiedlicher Bilder, Teilbilder, Bildsequenzen etc. handeln.

Die Bildgebungsvorrichtung 12a ist multimodal. Exemplarisch ist die Bildgebungsvorrichtung in drei grundlegenden Modi betreibbar, einem Multispektralmodus, einem Fluoreszenzmodus und einem Weißlichtmodus. Ferner kann vorgesehen sein, dass die Bildgebungsvorrichtung 12a zusätzlich oder alternativ zum Multispektralmodus in einem Hyperspektralmodus betreibbar ist. Die Beleuchtungsvorrichtung 10a ist multimodal. Die Beleuchtungsvorrichtung 10a ist in unterschiedlichen Beleuchtungsmodi betreibbar, in denen sie Licht für unterschiedliche Bildgebungsmodi liefert. Vorliegend ist die Beleuchtungsvorrichtung 10a in drei grundlegenden Modi betreibbar, einem Multispektralmodus, einem Fluoreszenzmodus und einem Weißlichtmodus. Ebenso ist das Bildgebungsgerät 11a in unterschiedlichen Betriebsmodi betreibbar, konkret ebenfalls zumindest in einem Multispektralmodus, einem Fluoreszenzmodus und einem Weißlichtmodus. Im entsprechenden Betriebsmodus der Bildgebungsvorrichtung 12a werden die Modi der Beleuchtungsvorrichtung 10a und/oder des Bildgebungsgeräts 11a aufeinander abgestimmt.

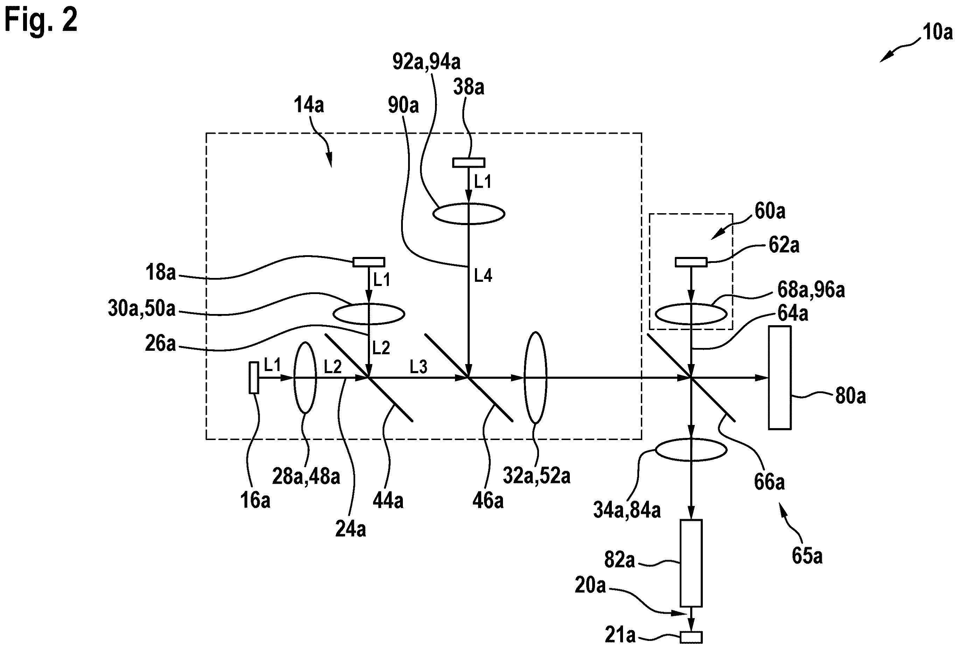

Fig. 2 zeigt eine schematische Darstellung der Beleuchtungsvorrichtung 10a. Die Beleuchtungsvorrichtung 10a umfasst eine Beleuchtungseinheit 14a, welche zu einer Bereitstellung einer Beleuchtung eingerichtet ist. Die Beleuchtungsvorrichtung 10a weist eine weitere Beleuchtungseinheit 60a auf, welche zu einer Bereitstellung einer weiteren Beleuchtung eingerichtet ist.

Die Beleuchtungsvorrichtung 10a weist eine Kombinationseinheit 65a mit einem Strahlteilerelement 66a auf, welches dazu eingerichtet ist, einen Teil der Beleuchtung und einen Teil der weiteren Beleuchtung zu einer Bildung einer Kombinationsbeleuchtung zusammenzuführen, die dann als Ausgangsbeleuchtung bereitgestellt wird.

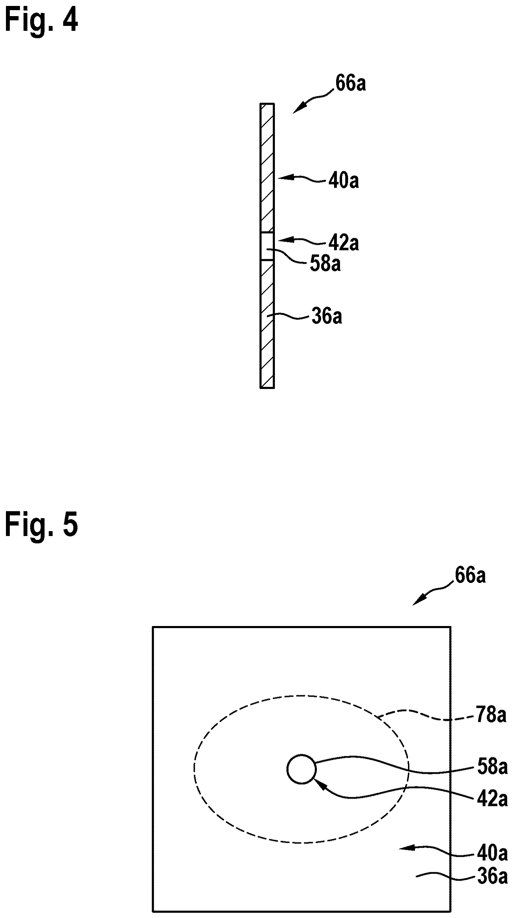

Das Strahlteilerelement 66a ist in Fig. 4 in einer seitlichen Schnittdarstellung und in Fig. 5 in einer Draufsicht gezeigt. Das Strahlteilerelement 66a weist einen für die Beleuchtung im Wesentlichen reflektierenden ersten Bereich 40a und einen für die weitere Beleuchtung im Wesentlichen transmittierenden zweiten Bereich 42a auf, welcher von dem ersten Bereich 40a vollständig umgeben ist. Das Strahlteilerelement 66a weist dabei einen flachen Breitbandspiegel 36a mit integrierender Lochblende 58a auf, welche den zweiten Bereich 42a definiert. Die Lochblende 58a ist durch eine senkrechte durchgehende Bohrung im Breitbandspiegel 36a gebildet.

Mittels des Strahlteilerelements 66a wird die Beleuchtung und die weitere Beleuchtung dadurch zusammengeführt, dass die Beleuchtung am Breitbandspiegel 36a reflektiert, die weitere Beleuchtung hingegen durch die Lochblende 58a transmittiert wird. Dabei wird die weitere Beleuchtung in der Lochblende 58a fokussiert. Durch das Strahlteilerelement 66a wird ein Beleuchtungsspektrum 54 der Beleuchtung und ein weiteres Beleuchtungsspektrum 56 der weiteren Beleuchtung jeweils im Wesentlichen unverändert zusammengeführt.

Die Beleuchtungsvorrichtung 10a weist eine Fokuslinse 84a hinter dem Strahlteilerelement 66a der Kombinationseinheit 65a in Richtung des Beleuchtungsausgangs 20a auf (vgl. Fig. 2). Mittels der Fokuslinse 84a wird die dem Beleuchtungsausgang 20a zugeführte Ausgangsbeleuchtung fokussiert. Die Beleuchtungsvorrichtung 10a kann zusätzlich einen Homogenisator 82a, beispielsweise in Form eines Homogenisatorstabs, zur Homogenisierung der Ausgangsbeleuchtung vor dem Beleuchtungsausgang 20a aufweisen, muss dies aber nicht.

Die Beleuchtungseinheit 14a weist eine erste Beleuchtungsquelle 16a, eine zweite Beleuchtungsquelle 18a und eine dritte Beleuchtungsquelle 38a auf. Alternativ könnte die Beleuchtungseinheit 14a auch mehr oder weniger Beleuchtungsquellen 16a, 18a, 38a aufweisen.

Die erste Beleuchtungsquelle 16a ist als eine LED ausgebildet. Die erste Beleuchtungsquelle 16a ist zur Emission von schmalbandigem Licht mit einer mittleren Wellenlänge von 940 nm eingerichtet.

Die zweite Beleuchtungsquelle 18a ist als eine LED ausgebildet. Die zweite Beleuchtungsquelle 18a ist zur Emission von schmalbandigem Licht mit einer mittleren Wellenlänge von 660 nm eingerichtet.

Die dritte Beleuchtungsquelle 38a ist als eine LED ausgebildet. Die dritte Beleuchtungsquelle 38a ist zur Emission von breitbandigem Licht mit Intensitätsmaxima bei 440 nm und 550 nm eingerichtet.

Die weitere Beleuchtungseinheit 60a weist eine weitere Beleuchtungsquelle 62a auf. Alternativ könnte die weitere Beleuchtungseinheit 60a auch mehrere weitere Beleuchtungsquellen 62a aufweisen, deren Strahlung mittels Strahlteilern und/oder Faser-Combinern der weiteren Beleuchtungseinheit 60a zusammengeführt sein kann.

Die weitere Beleuchtungsquelle 62a ist als eine Laserdiode ausgebildet. Die weitere Beleuchtungsquelle 62a ist zur Emission einer Laserlinie mit einer mittleren Wellenlänge von 470 nm, 640 nm oder 770 nm eingerichtet. Für den Fall mehrerer weiterer Beleuchtungsquellen 62a könnte die weitere Beleuchtungseinheit 60a für jede der genannten Wellenlängen eine eigene weitere Beleuchtungsquelle 62a aufweisen.

Die Beleuchtungsquellen 16a, 18a, 38a und die weitere Beleuchtungsquelle 62a sind zur Realisierung der unterschiedlichen Betriebsmodi unabhängig voneinander oder in Gruppen aktivierbar.

In alternativen Ausgestaltungen wären auch andere mittlere Wellenlängen und/oder Bandbreiten und/oder Spektren für die Beleuchtungsquellen 16a, 18a, 38a und die weitere Beleuchtungsquelle 62a denkbar.

Die Beleuchtungsvorrichtung 10a weist für jede Beleuchtungsquelle 16a, 18a, 38a der Beleuchtungseinheit 14a einen optischen Pfad 24a, 26a, 90a auf, welcher sich von der jeweiligen Beleuchtungsquelle 16a, 18a, 38a bis zum Beleuchtungsausgang 20a erstreckt.

Ein erster optischer Pfad 24a erstreckt sich von der ersten Beleuchtungsquelle 16a zum Beleuchtungsausgang 20a und umfasst genau drei optische Strahlformerelemente 28a, 32a, 34a.

Ein zweiter optischer Pfad 26a erstreckt sich von der zweiten Beleuchtungsquelle 18a zum Beleuchtungsausgang 20a und umfasst genau drei optische Strahlformerelemente 30a, 32a, 34a.

Ein dritter optischer Pfad 90a erstreckt sich von der dritten Beleuchtungsquelle 38a zum Beleuchtungsausgang 20a und umfasst genau drei optische Strahlformerelemente 32a, 34a, 92a.

Somit umfassen sämtliche optischen Pfade 24a, 26a, 90a von den Beleuchtungsquellen 16a, 18a, 38a der Beleuchtungseinheit 14a zum Beleuchtungsausgang 20a jeweils genau drei optische Strahlformerelemente 28a, 30a, 32a, 34a, 92a.

Die optischen Strahlformerelemente 28a, 30a, 92a sind als Kondensorlinsen 48a, 50a, 94a ausgebildet, und zwar als eine um eine Distanz L1 von der ersten Beleuchtungsquelle 16a beabstandete erste Kondensorlinse 48a, eine um eine Distanz L1 von der zweiten Beleuchtungsquelle 18a beabstandete zweite Kondensorlinse 50a und eine um eine Distanz L1 von der dritten Beleuchtungsquelle 38a beabstandete dritte Kondensorlinse 94a. Die erste Kondensorlinse 48a, die zweite Kondensorlinse 50a und die dritte Kondensorlinse 94a sind zueinander identisch.

Das optische Strahlformerelement 32a ist als eine gemeinsame Kompensatorlinse 52a ausgebildet, welche dem ersten optischen Pfad 24a, dem zweiten optischen Pfad 26a und dem vieren optischen Pfad 90a gemein ist.

Das optische Strahlformerelement 34a ist als die Fokuslinse 84a ausgebildet und allen optischen Pfaden 24a, 26a, 90a gemein.

Die Beleuchtungsvorrichtung 10a weist zwei Strahlteilerelemente 44a, 46a auf, welche jeweils dazu eingerichtet sind, eine der Beleuchtungsquellen 18a, 38a der Beleuchtungseinheit 14a in einen Strahlengang ausgehend von der Beleuchtungsquelle 16a in Richtung des Beleuchtungsausgangs 20a einzukoppeln.

Das Strahlteilerelement 44a ist Teil des ersten optischen Pfads 24a und des zweiten optischen Pfads 26a. Das Strahlteilerelement 44a ist jeweils um eine Distanz L2 von der ersten Kondensorlinse 48a und der zweiten Kondensorlinse 50a beabstandet. Das Strahlteilerelement 44a weist einen Kantenfilter ("edge filter") mit einer Kante auf, die im Wellenlängenraum zwischen einem von der ersten Beleuchtungsquelle 16a emittierten Wellenlängenbereich und einem von der zweiten Beleuchtungsquelle 18a emittierten Wellenlängenbereich liegt.

Das Strahlteilerelement 46a ist Teil des ersten optischen Pfads 24a, des zweiten optischen Pfads 26a und des dritten optischen Pfads 90a. Das Strahlteilerelement 46a ist um eine Distanz L4 von der dritten Kondensorlinse 94a beabstandet. Das Strahlteilerelement 46a weist einen Kantenfilter ("edge filter") mit einer Kante auf, die im Wellenlängenraum zwischen einem von der ersten Beleuchtungsquelle 16a und der zweiten Beleuchtungsquelle 18a emittierten Wellenlängenbereich und einem von der dritten Beleuchtungsquelle 38a emittierten Wellenlängenbereich liegt.

Die Strahlteilerelemente 44a, 46a sind optisch unmittelbar hintereinander angeordnet und um eine Distanz L3 voneinander beabstandet. Optisch zwischen dem Strahlteilerelement 46a und dem Strahlteilerelement 66a der Kombinationseinheit 65a ist die gemeinsame Kompensatorlinse 52a angeordnet.

Das Strahlteilerelement 66a der Kombinationseinheit 65a und gegebenenfalls der Homogenisator 82a sind Teil des ersten optischen Pfads 24a, des zweiten optischen Pfads 26a und des dritten optischen Pfads 90a.

Der erste optische Pfad 24a, der zweite optische Pfad 26a und der dritte optische Pfad 90a sind gleich lang, da zwischen den Distanzen L2, L3 und L4 folgender Zusammenhang gilt:

$$L4 = L2 + L3$$

Der erste optische Pfad 24a, der zweite optische Pfad 26a und der dritte optische Pfad 90a weisen zudem die gleichen optischen Strahlformerelemente 28a, 30a, 32a, 34a, 92a auf. Der erste optische Pfad 24a, der zweite optische Pfad 26a und der dritte optische Pfad 90a sind somit zueinander identisch.

Für jede der Beleuchtungsquellen 16a, 18a, 38a der Beleuchtungseinheit 14a ergibt sich demzufolge ein grundsätzlich ähnlicher Strahlengang zum Beleuchtungsausgang 20a: Der Strahlengang verläuft für jede der Beleuchtungsquellen 16a, 18a, 38a stets über die ihr zugeordnete Kondensorlinse 48a, 50a, 94a, über die gemeinsame Kompensatorlinse 52a und über die Fokuslinse 84a.

Die Beleuchtungsvorrichtung 10a weist für jede weitere Beleuchtungsquelle 62a der weiteren Beleuchtungseinheit 60a einen weiteren optischen Pfad 64a auf, im vorliegenden Fall lediglich einer, welcher sich von der jeweiligen weiteren Beleuchtungsquelle 62a bis zum Beleuchtungsausgang 20a erstreckt.

Der weitere optische Pfad 64a erstreckt sich von der weiteren Beleuchtungsquelle 62a zum Beleuchtungsausgang 20a und umfasst genau zwei optische Strahlformerelemente 34a, 68a.

Das optische Strahlformerelement 68a ist als eine Kondensorlinse 96a ausgebildet, und zwar als eine der weiteren Beleuchtungsquelle 62a zugeordnete weitere Kondensorlinse 96a. Die weitere Kondensorlinse 96a weicht von der ersten Kondensorlinse 48a, der zweiten Kondensorlinse 50a und der dritten Kondensorlinse 94a ab, könnte aber in alternativen Ausgestaltungen auch identisch sein.

Das optische Strahlformerelement 34a ist wiederum als die Fokuslinse 84 ausgebildet und ist mit den optischen Pfaden 24a, 26a, 90a gemein.

Da die weitere Beleuchtungsquelle 62a als eine starke Laserdiode ausgebildet ist und der weitere optische Pfad 64a relativ kurz ist, kann im weiteren optischen Pfad 64a auf eine Kompensatorlinse verzichtet werden. Die Strahlenverluste sind hier verkraftbar.

Der weitere optische Pfad 64a umfasst zudem das Strahlteilerelement 66a der Kombinationseinheit 65a und gegebenenfalls den Homogenisator 82a.

Die Beleuchtungseinheit 14a weist eine Optikeinheit 76a auf, welche dazu eingerichtet ist, die Beleuchtung am Strahlteilerelement 66a mit einem Strahlquerschnitt 78a bereitzustellen, welcher den zweiten Bereich 42a vollständig und den ersten Bereich 40a zumindest abschnittsweise überspannt. Dies ist in Fig. 5 nicht maßstäblich angedeutet. Dabei ist eine Fläche des Strahlquerschnitts 78a um zumindest einen Faktor 100 größer als eine Lichteintrittsfläche des zweiten Bereichs 42a. Die Optikeinheit 76a umfasst dabei die erste Kondensorlinse 48a, die zweite Kondensorlinse 50a, die dritte Kondensorlinse 94a und die gemeinsame Kompensatorlinse 52a.

Die weitere Beleuchtungseinheit 60a weist eine weitere Optikeinheit 98a auf, welche dazu eingerichtet ist, die weitere Beleuchtung so zu fokussieren, dass diese das Strahlteilerelement 66a lediglich innerhalb des zweiten Bereichs 42 passiert. Die weitere Optikeinheit 98a umfasst dabei die weitere Kondensorlinse 96a.

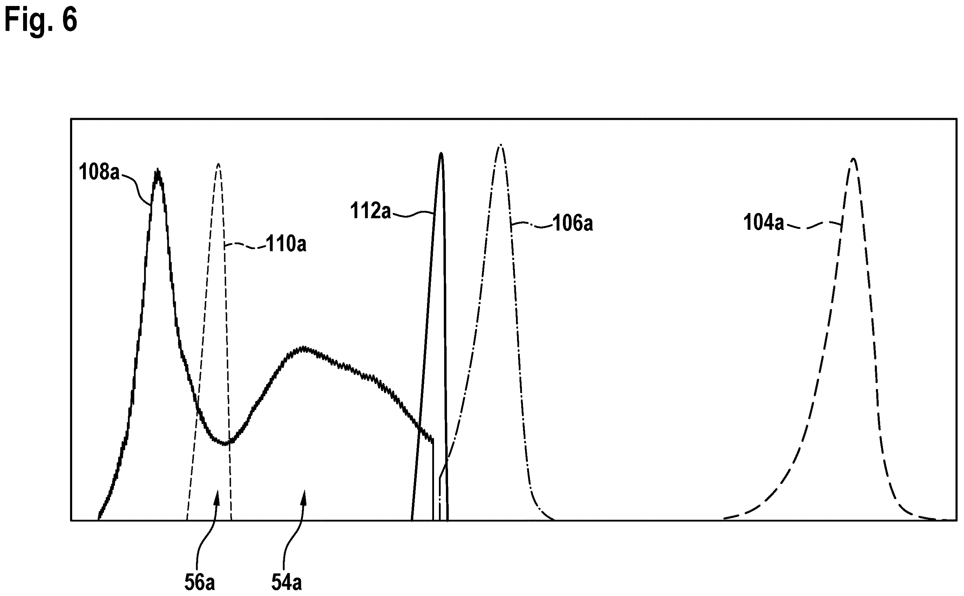

Fig. 6 zeigt ein Schaubild mit dem Beleuchtungsspektrum 54a und dem weiteren Beleuchtungsspektrum 56a.

Das Beleuchtungsspektrum 54a umfasst ein erstes Beleuchtungseinzelspektrum 104a, welches von der ersten Beleuchtungsquelle 16a stammt. Das Beleuchtungsspektrum 54a umfasst ein zweites Beleuchtungseinzelspektrum 106a, welches von der zweiten Beleuchtungsquelle 18a stammt. Das Beleuchtungsspektrum 54a umfasst ein drittes Beleuchtungseinzelspektrum 108a, welches von der dritten Beleuchtungsquelle 38a stammt. Die scharfen Schnitte im Beleuchtungsspektrum 54a zwischen dem zweiten Beleuchtungseinzelspektrum 106a und dem dritten Beleuchtungseinzelspektrum 108a sind dabei dem Strahlteilerelement 46a geschuldet.

Das weitere Beleuchtungsspektrum 56a umfasst ein weiteres erstes Beleuchtungseinzelspektrum 110a, welches von der weiteren Beleuchtungsquelle 62a stammt. In Fig. 6 ist zudem beispielhaft der Fall für eine weitere Beleuchtungseinheit 60a mit zwei weiteren Beleuchtungsquellen 62a gezeigt. Dann könnte das weitere Beleuchtungsspektrum 56a ein weiteres zweites Beleuchtungseinzelspektrum 112a aufweisen.

Das Beleuchtungsspektrum 54a ist breitbandiger ausgebildet als das weitere Beleuchtungsspektrum 56a. Insbesondere sind die Beleuchtungseinzelspektren 104a, 106a, 108a jeweils breitbandiger als die weiteren Beleuchtungseinzelspektren 110a, 112a. Zudem überlappt das Beleuchtungsspektrum 54a im Wellenlängenraum zumindest teilweise mit dem weiteren Beleuchtungsspektrum 56a. Insbesondere überlappt das dritte Beleuchtungseinzelspektrum 108a mit dem weiteren ersten Beleuchtungseinzelspektrum 110a und dem weiteren zweiten Beleuchtungseinzelspektrum 112a. Ferner überlappt das zweite Beleuchtungseinzelspektrum 106a mit dem weiteren zweiten Beleuchtungseinzelspektrum 112a.

Zurückkommend auf Fig. 2: Die Beleuchtungsvorrichtung 10a umfasst eine Detektoreinheit 80a zu einer Bestimmung zumindest einer Beleuchtungseigenschaft der Ausgangsbeleuchtung, und zwar der durch die Beleuchtungseinheit 14a bereitgestellten Beleuchtung. Durch die Lochblende 58a gelangt ein Teil der Beleuchtung auf den Detektor 80a und kann somit zu einer Überwachung der Beleuchtungseinheit 14a mittels des Detektors 80a herangezogen werden.

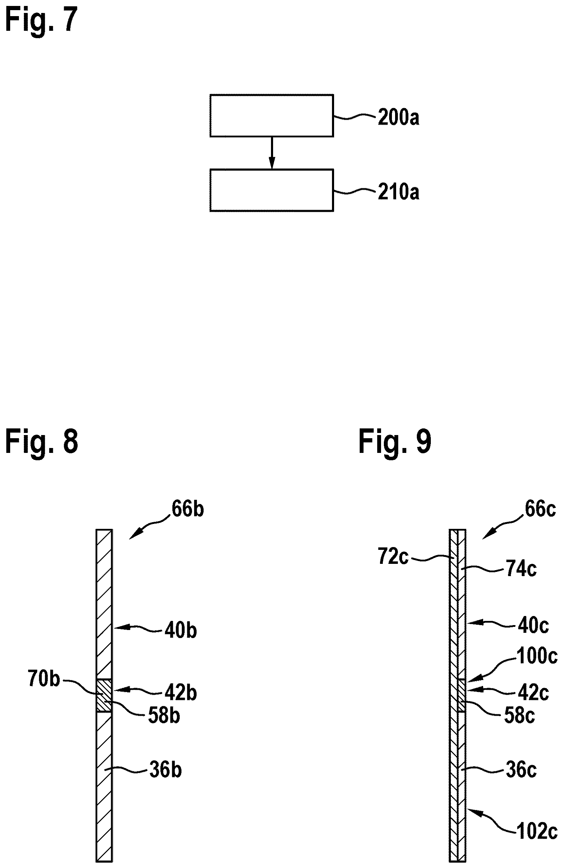

Fig. 7 zeigt ein Schaubild eines Verfahrens zum Betrieb der Beleuchtungsvorrichtung 10a. In einem Schritt 200a werden die Beleuchtungseinheit 14a und die weitere Beleuchtungseinheit 60a aktiviert. Dabei werden je nach Bedarf bestimmte der Beleuchtungsquellen 16a, 18a, 38a und weiteren Beleuchtungsquellen 62a aktiviert. Sofern sowohl eine der Beleuchtungsquellen 16a, 18a, 38a der Beleuchtungseinheit 14a als auch die weitere Beleuchtungsquelle 62a aktiviert ist, wird in einem Schritt 210a zumindest ein Teil der Beleuchtung und zumindest ein Teil der weiteren Beleuchtung zu einer Bildung einer Kombinationsbeleuchtung zusammengeführt. Dabei wird der Teil der Beleuchtung und der Teil der weiteren Beleuchtung zur Bildung der Kombinationsbeleuchtung mittels des Strahlteilerelements 66a zusammengeführt. Das Beleuchtungsspektrum 54a und das weitere Beleuchtungsspektrum 46a werden dabei jeweils im Wesentlichen unverändert zusammengeführt.

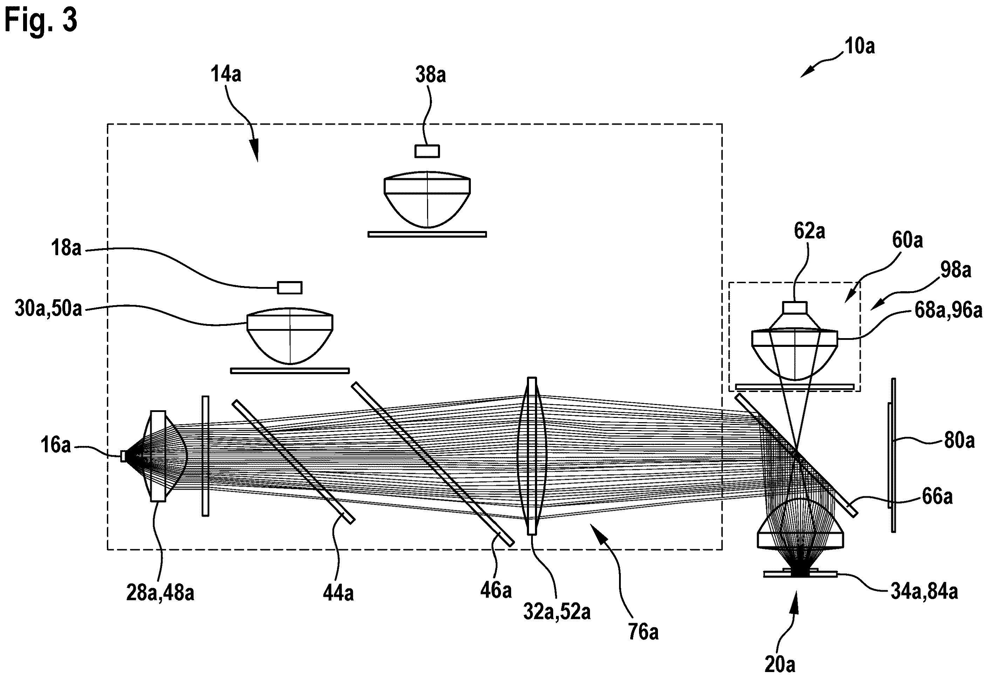

In den Fig. 8 und 9 sind weitere Ausführungsbeispiele der Erfindung gezeigt. Die folgende Beschreibung beschränkt sich im Wesentlichen auf die Unterschiede zwischen den Ausführungsbeispielen. Hinsichtlich von Baueinheiten und Bauteilen mit gleichen Bezugszeichen kann grundsätzlich auf die Beschreibung der anderen Ausführungsbeispiele, insbesondere des Ausführungsbeispiels der Fig. 1 bis 7, verwiesen werden. Zur Unterscheidung ist den Bezugzeichen der Ausführungsbeispiele der Fig. 8 und 9, anstatt des Buchstabens "a" des Ausführungsbeispiels der Fig. 1 bis 7, einer der Buchstaben "b" oder "c" nachgestellt.

Das Ausführungsbeispiel der Fig. 8 unterscheidet sich vom vorherigen Ausführungsbeispiel dadurch, dass ein Strahlteilerelement 66b in einem zweiten Bereich 42b ein optisches Filterelement 70b aufweist. Dieses optische Filterelement 70b ist passgenau in eine Lochblende 58b eines Breitbandspiegels 36b des Strahlteilerelement 66b eingepasst und insbesondere eingeklebt.

Über einen gesamten Wellenlängenbereich der Beleuchtung und der weiteren Beleuchtung reflektiert das optische Filterelement 70b einen geringen Anteil von maximal 1 % einer auftreffenden Lichtintensität und transmittiert im Übrigen.

Damit kann mit Bezug auf das vorherige Ausführungsbeispiel mittels der Detektoreinheit 80a eine Überwachung der Beleuchtungseinheit 14a und der weiteren Beleuchtungseinheit 60a erfolgen, und zwar sowohl gemeinsam in einem gemeinsamen Betrieb der Beleuchtungseinheit 14a und der weiteren Beleuchtungseinheit 60a als auch einzeln bei sequentieller Aktivierung der Beleuchtungseinheit 14a und der weiteren Beleuchtungseinheit 60a nacheinander.

Im Ausführungsbeispiel gemäß Fig. 9 weist ein Strahlteilerelement 66c ein für die Beleuchtung und die weitere Beleuchtung im Wesentlichen transmittierendes Substrat 72c mit einer Beschichtung 74c auf, welche in einem ersten Bereich 40c breitbandig reflektiert und welche in einem zweiten Bereich 42c für die Beleuchtung teilweise reflektierend und für die weitere Beleuchtung teilweise transmittierend ausgebildet ist.

Die Beschichtung 74c weist im ersten Bereich 40c einen erster Beschichtungsabschnitt 100c und im zweiten Bereich 42c einen zweiten Beschichtungsabschnitt 102c auf. Dabei ist der erste Beschichtungsabschnitt 100c bezüglich einer Hauptoberfläche des Substrats 72c neben dem zweiten Beschichtungsabschnitt 102c angeordnet und umschließt diesen vollständig. Der zweite Beschichtungsabschnitt 102c ist kreisscheibenförmig ausgebildet.

Die Beschichtung 74c bildet dabei im zweiten Bereich 42c einen optischen Filter aus. Hinsichtlich von Filtereigenschaften des Filters kann auf die Filtereigenschaftes des optischen Filterelements 70b aus dem vorherigen Ausführungsbeispiel verweisen werden.

## Bezugszeichenliste

| 10 | Beleuchtungsvorrichtung |
| 11 | Bildgebungsgerät |
| 12 | Bildgebungsvorrichtung |
| 14 | Beleuchtungseinheit |
| 16 | erste Beleuchtungsquelle |
| 18 | zweite Beleuchtungsquelle |
| 20 | Beleuchtungsausgang |
| 21 | optische Schnittstelle |
| 22 | Lichtleiter |
| 24 | erster optischer Pfad |
| 26 | zweiter optischer Pfad |
| 28 | optisches Strahlformerelement |
| 30 | optisches Strahlformerelement |
| 32 | optisches Strahlformerelement |
| 34 | optisches Strahlformerelement |
| 36 | Breitbandspiegel |
| 38 | dritte Beleuchtungsquelle |
| 40 | erster Bereich |
| 42 | zweiter Bereich |
| 44 | Strahlteilerelement |

| 46 | Strahlteilerelement |
|----|----|
| 48 | erste Kondensorlinse |
| 50 | zweite Kondensorlinse |
| 52 | gemeinsame Kompensatorlinse |
| 54 | Beleuchtungsspektrum |
| 56 | weiteres Beleuchtungsspektrum |
| 58 | Lochblende |
| 60 | weitere Beleuchtungseinheit |
| 62 | weitere Beleuchtungsquelle |
| 64 | weiterer optischer Pfad |
| 65 | Kombinationseinheit |
| 66 | Strahlteilerelement |
| 68 | optisches Strahlformerelement |
| 70 | Optisches Filterelement |
| 72 | Substrat |
| 74 | Beschichtung |
| 76 | Optikeinheit |
| 78 | Strahlquerschnitt |
| 80 | Detektoreinheit |
| 82 | Homogenisator |
| 84 | Fokuslinse |
| 86 | Kavität |
| 88 | Anzeigeeinheit |
| 90 | dritter optischer Pfad |
| 92 | optisches Strahlformerelement |
| 94 | dritte Kondensorlinse |
| 96 | weitere Kondensorlinse |
| 98 | weitere Optikeinheit |
| 100 | erster Beschichtungsabschnitt |
| 102 | zweiter Beschichtungsabschnitt |
| 104 | erstes Beleuchtungseinzelspektrum |
| 106 | zweites Beleuchtungseinzelspektrum |
| 108 | drittes Beleuchtungseinzelspektrum |
| 110 | weiteres erstes Beleuchtungseinzelspektrum |
| 112 | weiteres zweites Beleuchtungseinzelspektrum |
| 200 | Schritt |
| 210 | Schritt |

**Patentansprüche**

1. Beleuchtungsvorrichtung (10) für ein medizinisches Bildgebungsgerät (11) wie ein Endoskop, Exoskop und/oder Mikroskop, umfassend:

    - eine Beleuchtungseinheit (14), welche zu einer Bereitstellung einer Beleuchtung mit einem Beleuchtungsspektrum (54) eingerichtet ist,
    - eine weitere Beleuchtungseinheit (60), welche zu einer Bereitstellung einer weiteren Beleuchtung mit einem weiteren Beleuchtungsspektrum (56) eingerichtet ist, und
    - eine Kombinationseinheit (65), welche dazu eingerichtet ist, zumindest einen Teil der Beleuchtung und zumindest einen Teil der weiteren Beleuchtung zu einer Bildung einer Kombinationsbeleuchtung zusammenzuführen,

**dadurch gekennzeichnet, dass**

die Kombinationseinheit (65) ein Strahlteilerelement (66) aufweist, welches einen für die Beleuchtung im Wesentlichen reflektierenden ersten Bereich (40) und einen für die weitere Beleuchtung im Wesentlichen transmittierenden zweiten Bereich (42) aufweist, welcher neben dem ersten Bereich (40) angeordnet und bevorzugt von dem ersten Bereich (40) zumindest teilweise umgeben ist.

2. Beleuchtungsvorrichtung (10) nach Anspruch 1,
**dadurch gekennzeichnet, dass**
das Beleuchtungsspektrum (54) im Wellenlängenraum zumindest teilweise mit dem weiteren Beleuchtungsspektrum (56) überlappt.

3. Beleuchtungsvorrichtung (10) nach einem der vorhergehenden Ansprüche,
**dadurch gekennzeichnet, dass**
das Beleuchtungsspektrum (54) breitbandiger ausgebildet ist als das weitere Beleuchtungsspektrum (56).

4. Beleuchtungsvorrichtung (10) nach einem der vorhergehenden Ansprüche,
**dadurch gekennzeichnet, dass**
zumindest eine Beleuchtungsquelle (16, 18, 38) der Beleuchtungseinheit (14) eine LED umfasst.

5. Beleuchtungsvorrichtung (10) nach einem der vorhergehenden Ansprüche,
**dadurch gekennzeichnet, dass**
zumindest eine weitere Beleuchtungsquelle (62) der weiteren Beleuchtungseinheit (60) einen Laser oder eine Laserdiode umfasst.

6. Beleuchtungsvorrichtung (10) nach einem der vorhergehenden Ansprüche,
**dadurch gekennzeichnet, dass**
der zweite Bereich (42) für die Beleuchtung zumindest teilweise transmittierend ist.

7. Beleuchtungsvorrichtung (10) nach einem der vorhergehenden Ansprüche,
**dadurch gekennzeichnet, dass**
der zweite Bereich (42) für die weitere Beleuchtung zumindest teilweise reflektierend ist.

8. Beleuchtungsvorrichtung (10) nach einem der vorhergehenden Ansprüche,
**dadurch gekennzeichnet, dass**
das Strahlteilerelement (66) der Kombinationseinheit (65) einen Breitbandspiegel (36) mit integrierender Lochblende (58) umfasst, welche den zweiten Bereich (42) definiert.

9. Beleuchtungsvorrichtung (10) nach Anspruch 8,
**dadurch gekennzeichnet, dass**
das Strahlteilerelement (66) der Kombinationseinheit (65) im zweiten Bereich (42) ein optisches Filterelement (70) aufweist.

10. Beleuchtungsvorrichtung (10) nach einem der vorhergehenden Ansprüche,
**dadurch gekennzeichnet, dass**
das Strahlteilerelement (66) der Kombinationseinheit (65) ein für die Beleuchtung und die weitere Beleuchtung im Wesentlichen transmittierendes Substrat (72) und eine Beschichtung (74) auf dem Substrat (72) aufweist, welche im ersten Bereich (40) breitbandig reflektiert und welche im zweiten Bereich (42) für die weitere Beleuchtung zumindest teilweise transmittierend ausgebildet ist.

11. Beleuchtungsvorrichtung (10) nach Anspruch 10,
**dadurch gekennzeichnet, dass**
die Beschichtung (74) im zweiten Bereich (42) einen optischen Filter bildet.

12. Beleuchtungsvorrichtung (10) nach einem der vorhergehenden Ansprüche,
**dadurch gekennzeichnet, dass**
die Beleuchtungseinheit (14) eine Optikeinheit (76) aufweist, welche dazu eingerichtet ist, die Beleuchtung am Strahlteilerelement (66) der Kombinationseinheit (65) mit einem Strahlquerschnitt (78) bereitzustellen, welcher den zweiten Bereich (42) vollständig und den ersten Bereich (40) zumindest abschnittsweise überspannt.

13. Beleuchtungsvorrichtung (10) nach Anspruch 12,
**dadurch gekennzeichnet, dass**
eine Fläche des Strahlquerschnitts (78) um zumindest einen Faktor 2 größer ist als eine Lichteintrittsfläche des zweiten Bereichs (42).

14. Beleuchtungsvorrichtung (10) nach einem der vorhergehenden Ansprüche,
**dadurch gekennzeichnet, dass**
die weitere Beleuchtungseinheit (60) eine weitere Optikeinheit (98) aufweist, welche dazu eingerichtet ist, die weitere Beleuchtung so zu fokussieren, dass diese das Strahlteilerelement (66) der Kombinationseinheit (65) lediglich innerhalb des zweiten Bereichs (42) passiert.

15. Beleuchtungsvorrichtung (10) nach einem der vorhergehenden Ansprüche,
**gekennzeichnet durch**

einen Beleuchtungsausgang (20), welcher eine optische Schnittstelle (21) aufweist und zu einer Bereitstellung einer Ausgangsbeleuchtung eingerichtet ist, welche zumindest teilweise durch die Kombinationsbeleuchtung gebildet ist, und eine Fokuslinse (84), welche dazu eingerichtet ist, die Kombinationsbeleuchtung auf die optische Schnittstelle (21) zu fokussieren.

16. Beleuchtungsvorrichtung (10) nach einem der vorhergehenden Ansprüche,
**dadurch gekennzeichnet, dass**

das Beleuchtungsspektrum (54) im Wellenlängenraum zumindest teilweise mit dem weiteren Beleuchtungsspektrum (56) überlappt, wobei die Kombinationseinheit (65) dazu eingerichtet ist, das Beleuchtungsspektrum (54) und das weitere Beleuchtungsspektrum (56) jeweils im Wesentlichen unverändert zusammenzuführen.

17. Beleuchtungsvorrichtung (10) nach einem der vorhergehenden Ansprüche, **dadurch gekennzeichnet, dass**
die Beleuchtungsvorrichtung (10) eine Detektoreinheit (80) zur Bestimmung zumindest einer Beleuchtungseigenschaft der Ausgangsbeleuchtung und/oder der Kombinationsbeleuchtung umfasst.

18. Medizinische Bildgebungsvorrichtung (12), insbesondere endoskopische, exoskopische und/oder mikroskopische medizinische Bildgebungsvorrichtung (12), mit einer Beleuchtungsvorrichtung (10) nach einem der vorhergehenden Ansprüche.

19. Verfahren zum Betrieb einer Beleuchtungsvorrichtung (10) nach einem der Ansprüche 1 bis 17, wobei die Beleuchtungsvorrichtung (10) umfasst:

- Bereitstellen einer Beleuchtungseinheit (14), welche zur Bereitstellung einer Beleuchtung mit einem Beleuchtungsspektrum (54) eingerichtet ist,
- Bereitstellen einer weiteren Beleuchtungseinheit (60), welche zu einer Bereitstellung einer weiteren Beleuchtung mit einem weiteren Beleuchtungsspektrum (56) eingerichtet ist,
- Zusammenführen zumindest eines Teils der Beleuchtung und zumindest eines Teils der weiteren Beleuchtung zur Bildung einer Kombinationsbeleuchtung,

**gekennzeichnet durch** die weiteren Schritte:

- Zusammenführen des Teils der Beleuchtung und des Teils der weiteren Beleuchtung zur Bildung der Kombinationsbeleuchtung mittels eines Strahlteilerelements (56), welches einen für die Beleuchtung im Wesentlichen reflektierenden ersten Bereich (40) und einen für die weitere Beleuchtung im Wesentlichen transmittierenden zweiten Bereich (42) aufweist, welcher neben dem ersten Bereich (40) angeordnet und bevorzugt von dem ersten Bereich (40) zumindest teilweise umgeben ist.

20. Verfahren nach Anspruch 19,
**dadurch gekennzeichnet, dass**

das Beleuchtungsspektrum (54) im Wellenlängenraum zumindest teilweise mit dem weiteren Beleuchtungsspektrum (56) überlappt, wobei das Beleuchtungsspektrum (54) und das weitere Beleuchtungsspektrum (46) jeweils im Wesentlichen unverändert zusammengeführt werden.

# Fig. 1

**Fig. 2**

EP 4 706 497 A1

Fig. 3

# Fig. 4

66a

40a

42a
58a

36a

# Fig. 5

66a

78a

58a

42a

40a
36a

Fig. 6

# Fig. 7

```
┌─────────────────┐
│                 │─── 200a
└─────────────────┘
         │
         ▼
┌─────────────────┐
│                 │─── 210a
└─────────────────┘
```

# Fig. 8

66b
40b
70b
42b
58b
36b

# Fig. 9

66c
72c
74c
40c
100c
42c
58c
36c
102c

Europäisches Patentamt
European Patent Office
Office européen des brevets

**EUROPÄISCHER RECHERCHENBERICHT**

Nummer der Anmeldung

EP 25 19 7318

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 11 2015 002455 T5 (OLYMPUS CORP [JP]) 23. März 2017 (2017-03-23)<br><br>* Absätze [0021] - [0045], [0061], [0071], [0072]; Abbildungen 1-3,8 *<br>----- | 1-8, 12-16, 18-20 | INV.<br>A61B1/06<br>A61B1/07<br>G02B23/24<br>G02B27/10<br>G02B27/14 |
| X | WO 2024/037590 A1 (CHANGZHOU UNITED IMAGING HEALTHCARE SURGICAL TECH CO LTD [CN]) 22. Februar 2024 (2024-02-22) * Absätze [0023] - [0037], [0080] - [0101]; Abbildungen 2,15 * | 1-7,9-20 | |
| X,P | & EP 4 555 915 A1 (CHANGZHOU UNITED IMAGING SURGICAL CO LTD [CN]) 21. Mai 2025 (2025-05-21) * Absätze [0023] - [0037], [0080] - [0101]; Abbildungen 2,15 *<br>----- | 1-7,9-20 | |
| X | DE 692 12 382 T2 (XILLIX TECHNOLOGIES CORP [CA]) 6. März 1997 (1997-03-06)<br><br>* Seite 8, Zeile 1 - Seite 9, Zeile 3; Abbildung 3 *<br>----- | 1-3,5,6, 8,12-16, 18-20 | |
| X | US 2005/013132 A1 (KIM DAE-SIK [KR] ET AL) 20. Januar 2005 (2005-01-20)<br><br>* Absätze [0041], [0057] - [0061]; Abbildungen 20-22 *<br>----- | 1-3,5,6, 8,12-16, 18-20 | |

RECHERCHIERTE SACHGEBIETE (IPC)

A61B
G02B

Der vorliegende Recherchenbericht wurde für alle Patentansprüche erstellt

| Recherchenort | Abschlußdatum der Recherche | Prüfer |
|---|---|---|
| München | 9. Dezember 2025 | Rick, Kai |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 25 19 7318

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-12-2025

| Im Recherchenbericht angeführtes Patentdokument | Datum der Veröffentlichung | Mitglied(er) der Patentfamilie | Datum der Veröffentlichung |
|---|---|---|---|
| DE 112015002455 T5 | 23-03-2017 | CN 106413521 A | 15-02-2017 |
| | | DE 112015002455 T5 | 23-03-2017 |
| | | JP 6314041 B2 | 18-04-2018 |
| | | JP 2016002302 A | 12-01-2016 |
| | | US 2017095144 A1 | 06-04-2017 |
| | | WO 2015194312 A1 | 23-12-2015 |
| WO 2024037590 A1 | 22-02-2024 | EP 4555915 A1 | 21-05-2025 |
| | | WO 2024037590 A1 | 22-02-2024 |
| DE 69212382 T2 | 06-03-1997 | AT E140600 T1 | 15-08-1996 |
| | | CA 2042075 A1 | 09-11-1992 |
| | | DE 69212382 T2 | 06-03-1997 |
| | | EP 0512965 A1 | 11-11-1992 |
| | | JP 3317409 B2 | 26-08-2002 |
| | | JP H0654792 A | 01-03-1994 |
| | | US 5507287 A | 16-04-1996 |
| US 2005013132 A1 | 20-01-2005 | CN 1591168 A | 09-03-2005 |
| | | KR 20040102301 A | 04-12-2004 |
| | | US 2005013132 A1 | 20-01-2005 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 202014010558 U1
- DE 102020105458 A1
- US 10481095 B2
- US 11668922 B2